(19) **Europäisches Patentamt** / **European Patent Office** / **Office européen des brevets**

(11) **EP 2 877 135 B1**

(12) **EUROPEAN PATENT SPECIFICATION**

(45) Date of publication and mention of the grant of the patent:
**20.06.2018 Bulletin 2018/25**

(21) Application number: **12795217.4**

(22) Date of filing: **15.11.2012**

(51) Int Cl.:
*A61F 13/00* (2006.01)    *A61F 13/02* (2006.01)
*A61L 15/16* (2006.01)    *A61L 15/42* (2006.01)
*A61L 15/58* (2006.01)

(86) International application number:
**PCT/US2012/065196**

(87) International publication number:
**WO 2014/018077 (30.01.2014 Gazette 2014/05)**

(54) **SILICONE/ACRYLIC HYBRID ADHESIVES**

SILICONE/ACRYL HYBRIDKLEBSTOFFE

ADHÉSIFS HYBRIDES À BASE DE SILICONE/ACRYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2012 US 201213556867**

(43) Date of publication of application:
**03.06.2015 Bulletin 2015/23**

(73) Proprietor: **Avery Dennison Corporation Pasadena, CA 91103 (US)**

(72) Inventors:
- **CARTY, Neal**
  **Glendale, California 91203 (US)**
- **IYER, Pradeep**
  **Glendale, California 91203 (US)**
- **PARIS, Timothy, L.**
  **Glendale, California 91203 (US)**
- **DEHLINGER, Anne, M.**
  **Glendale, California 91203 (US)**
- **MOORE, Michael, J.**
  **Glendale, California 91203 (US)**
- **BECKER, William**
  **Glendale, California 91203 (US)**

(74) Representative: **Müller-Boré & Partner Patentanwälte PartG mbB Friedenheimer Brücke 21 80639 München (DE)**

(56) References cited:
EP-A1- 2 123 728        EP-A2- 1 184 039
WO-A2-2010/124187      US-A- 5 907 018

**Description**

The present application is a Continuation-In-Part of U.S. Application No. 13/556,867 filed July 24, 2012, which is a Continuation-In-Part of U.S. Application No. 13/270,631 filed October 11, 2011, which is a Continuation-In-Part of U.S. Application No. 13/021,873 filed February 7, 2011, which claims the benefit of U.S. Provisional Application No. 61/302,593 filed February 9, 2010.

The present subject matter relates to multilayer articles using silicone/acrylic hybrid adhesives such as a sheet assembly containing a number of removable pre-cut adhesive strips for use in the healthcare field. The sheet assembly utilizes the silicone/acrylic blend adhesive and carries strips that can be easily removed from a substrate upon exposure to a debonding agent.

The treatment of individuals in various healthcare related environments typically requires the use of a wide variety of medical devices, appliances, pharmaceuticals and other treatments. In order to reduce the possible spread of disease, or contamination within the surgical or treatment theaters, typically, devices are not reused and must be disposed of once the patient or individual has been treated.

Certain treatment devices, such as intravenous or IV bags, oxygen and nutritional supply tubes, catheters, monitoring device feed lines and the like require that the device be removably attached to the patient or the individual. Commonly, a piece of tape, which is often supplied from a roll, is torn from the roll supply and then applied to the tube, line or other device to secure the device to the patient. In an effort to reduce possible contamination, the remaining portion of the roll of tape is typically discarded leading to additional waste and cost in the care facility. When multiple tape sizes are required to hold or secure different appliances to the patient, then different sized rolls of tape are needed, which as noted are discarded after exposure to the treatment theater, thereby leading to additional waste and expense.

Accordingly, in view of these practices, a need remains for an alternate strategy by which adhesive strips and particularly those having different sizes, can be provided and maintained for convenient access by a healthcare practitioner.

Adhesives are widely used for securing a variety of articles to substrate(s). Pressure sensitive adhesives (PSA's), in particular, have been widely studied in an attempt to tailor their properties so that they readily "wet out" and bond rapidly to a given surface upon application of minimal pressure. Often, the goal is that articles carrying these adhesives eventually debond, with equal ease, from an underlying surface cleanly, i.e. without leaving any residue behind from either cohesive and/or facestock-adhesive interfacial failures. This objective entails balancing a number of seemingly contradictory properties at the adhesive interface including (i) optimizing the viscoelastic performance window of the adhesive, (ii) assessing the chemistry and solubility parameters of the adhesive components involved and whether they are single or multi-phase separated in nature, (iii) determining the extent of appropriate crosslinking, (iv) considering the conditions of bonding, e.g. pressure, surface roughness, etc, (v) assessing the application and dwell conditions of the adhesive, e.g. contact area and time, temperature, pressure, environmental conditions, etc., and (vi) addressing debonding modes between the adhesive and substrate, e.g. peel angle, speed, environmental conditions, etc.

Although adhesive debonding has been widely discussed and recognized to be an important challenge, few solutions have been achieved. The challenges associated with adhesive debonding are well recognized. Adhesive debonding and particularly, with ease and on demand, still presents a formidable technological hurdle.

A wide array of medical products are designed to attach securely to skin and to be retained thereto under a range of adverse conditions including contact with water, e.g. as may be encountered during bathing, swimming, etc.; contact with sweat, sebum or other body fluids; adhering to uneven or complex surface(s) associated with the skin or body that deform variably under mechanical stresses; exposure to heat, e.g. as may occur during a sauna; exposure to sunlight or other environmental factors; contact with other liquids such as hot or cold beverages; and/or being subjected to physical stresses resulting from motion such as during exercise. In view of these and other factors, adhesives for medical applications are typically engineered to adhere securely and for extended time periods to skin.

While many commercial products are known that purportedly facilitate removing an adhesively adhered article from a user's skin, there remains a critical and unmet need for ready, painless removal of the article, on demand, and without causing trauma. This need is particularly evident when adhesively adhered products are peeled off from the skin of elderly patients which is typically fragile and thin. In addition, a need exists for readily removable articles that can be used with children, cancer patients and specifically those with skin cancer, premature babies that have skin that is not fully developed, those with diseases that have a severe impact on the skin, or sensitive skin.

Accordingly, despite the numerous and varied attempts of prior artisans, a need remains for a strategy by which an adhesively adhered article may be easily and painlessly removed from a user's skin, without causing trauma and without any detrimental effects upon the adhesive or the article prior to removal. More particularly, a need remains for adhesives, articles, systems or kits, methods and materials for achieving this unique feature.

The difficulties and drawbacks associated with previously known adhesive articles and practices are overcome in the present subject matter as follows.

In one aspect of the present subject matter, a multilayer article is provided having a plurality of adhesive strips provided on a release liner. The strips are adapted to be easily removed after application to a surface of interest by exposure to a debonding agent. The article comprises a release liner defining a release face, a substrate layer having at least one cut line extending across its thickness and defining a plurality of adhesive strips, at least one adhesive layer extending between the release liner and the substrate layer, the adhesive layer including a pressure sensitive adhesive including (i) at least one silicone containing component, and (ii) at least one acrylic polymer, and an interior flow control layer disposed between the release liner and the substrate layer. The flow control layer defines a plurality of conduits or apertures from one face of the layer through the thickness of the layer to the other oppositely directed face of the layer so as to exhibit a controllable flow profile such that upon exposure to a debonding agent, the debonding agent is readily transported to the adhesive layer, wherein the debonding agent is an agent or combination of agents that serve to reduce and preferably eliminate an adhesive bond between the adhesive and the surface of interest.

In another aspect, the present subject matter provides a kit comprising a multilayer article as described above, and a debonding agent.

In still another aspect, the present subject matter provides a method of facilitating removal of an article from a surface after the article is adhesively attached thereto. The article includes a carrier sheet, at least one adhesive layer disposed along the carrier sheet, the adhesive layer including a pressure sensitive adhesive including (i) at least one silicone containing component, and (ii) at least one acrylic polymer, a substrate layer defining at least one cut line extending across its thickness, and a flow control layer disposed between the at least one adhesive layer and the substrate layer. The flow control layer exhibits a controllable flow profile. The method comprises exposing the article to an effective amount of at least one debonding agent to thereby debond the adhesively attached article from the surface.

In yet another aspect, the present subject matter provides a method for adhesively applying an article to skin and reducing discomfort associated with removal of the article from the skin. The method comprises providing an article including a carrier sheet, at least one adhesive layer disposed along the carrier sheet, the adhesive layer including a pressure sensitive adhesive including (i) at least one silicone containing component, and (ii) at least one acrylic polymer, a substrate layer defining at least one cut line extending across its thickness, and a flow control layer disposed between the at least one adhesive layer and the substrate layer. The flow control layer exhibits a controllable flow profile. The method also comprises adhesively applying the article to skin by contacting at least a portion of the pressure sensitive adhesive to the skin to thereby adhesively adhere the article to the skin. And, the method additionally comprises exposing the article to an effective amount of at least one debonding agent to thereby debond the adhesively attached article from the skin.

As will be realized, the subject matter described herein is capable of other and different embodiments and its several details are capable of modifications in various respects, all without departing from the claimed subject matter. Accordingly, the drawings and description are to be regarded as illustrative and not restrictive.

Figure 1 is a graph of peel force reduction for various samples evaluated in Example 1.
Figure 2 is a graph of peel force reduction for various samples evaluated in Example 1.
Figure 3 is a graph of peel force reduction for various samples evaluated in Example 1.
Figure 4 is a graph of peel force reduction for various samples evaluated in Example 1.
Figure 5 is a graph of peel force reduction for various samples evaluated in Example 1.
Figure 6 is a planar view of an outer face of one version of a sheeted medical tape article utilizing fluid activated debonding.
Figure 7 is an exploded schematic view of the sheeted article depicted in Figure 6.
Figure 8 is a schematic unexploded cross sectional view of the sheeted article of Figure 7 taken along line III-III in Figure 7.
Figure 9 is a schematic unexploded cross sectional view of an alternative version of the sheeted article of Figure 7 taken along line IV-IV in Figure 7.
Figure 10 is a schematic unexploded view of an edge region of the sheeted article of Figure 7 taken along line V-V in Figure 7.
Figure 11 is a schematic unexploded view similar to Figure 5 depicting a finger-lift feature provided in the sheeted article of Figure 7.
Figure 12 is a schematic unexploded view similar to Figure 10 depicting use of an adhesive overlay strip in the sheeted article of Figure 7.
Figure 13 is a schematic unexploded view similar to Figure 10 depicting a finger-lift feature and an adhesive overlay strip in the sheeted article of Figure 7.
Figure 14 is a planar view of an outer face of another version of a sheeted medical tape article utilizing fluid activated debonding.
Figure 15 is an exploded schematic view of the sheeted article depicted in Figure 14.
Figure 16 is a schematic unexploded cross sectional view of the sheeted article of Figure 15 taken along line XI-XI in Figure 15.

Figure 17 is a schematic unexploded cross sectional view of an alternative version of the sheeted article of Figure 15 taken along line XII-XII in Figure 15.

Figure 18 is a schematic unexploded view of an edge region of the sheeted article of Figure 15 taken along line XIII-XIII in Figure 15.

Figure 19 is a schematic unexploded view similar to Figure 18 depicting a finger-lift feature provided in the sheeted article of Figure 15.

Figure 20 is a schematic unexploded view similar to Figure 18 depicting use of an adhesive overlay strip in the sheeted article of Figure 15.

Figure 21 is a schematic unexploded view similar to Figure 18 depicting a finger-lift feature and an adhesive overlay strip in the sheeted article of Figure 15.

Figure 22 is a graph of peel force for two sets of tape samples evaluated and described herein.

The present subject matter relates to sheeted medical adhesive articles utilizing fluid activated debonding and particularly articles using the adhesives described herein. The articles are typically in the form of strips which are pre-cut and reside on a carrier sheet. The articles include an outer substrate or facestock which may optionally be adapted to receive indicia, markings, or other information or designs. The articles also include one or more adhesive layers for attaching the article, e.g. a strip, to a surface of interest, such as for example, human skin. The articles additionally include a flow control layer generally within an interior region of the article and between the substrate or facestock layer, and an adhesive layer. In many versions of the sheeted articles, a carrier sheet is provided alongside an outermost face of an adhesive layer to protect the adhesive face until application. The carrier sheet also serves to support the substrate or facestock layer particularly when printing thereon, and to impart rigidity to the sheeted medical article prior to removal from the sheet and application to a surface of interest. After application to a surface of interest, the article or strip can be easily removed by application of a fluid debonding agent. In certain versions, the adhesive used is a pressure sensitive adhesive comprising a minority proportion of one or more silicone/acrylic copolymers and a majority proportion of one or more acrylic polymers. The articles may optionally include various features such as a finger-lift feature and/or utilize one or more adhesive overlay strips. These and other aspects are described in greater detail as follows.

Adhesive

Pressure sensitive adhesives suitable for use with the present subject matter include permanent, removable, repositionable or other adhesives or combinations thereof that may meet the particular needs of the end use application. For example, a removable adhesive may be used to secure a section of medical tubing to a patient's arm such as with an intravenous (IV) tube. It should be understood that the multilayer laminate or assembly can be provided with a single type of adhesive, or alternatively, different types of adhesive can be applied to the sheet article in order to serve different functions. For example, certain removable strips (which will be described herein) can be provided with a permanent adhesive and other types of removable strips can be provided with a removable adhesive. Additional details and aspects of adhesives are provided herein.

In one version of the present subject matter, a particular type of adhesive is used for a wide array of medical articles such as for example the strips described herein. Although a wide array of pressure sensitive adhesives can be used in the present subject matter, a pressure sensitive adhesive comprising a blend of one or more silicone components and one or more acrylic components has been identified as providing numerous benefits. In a particular version, the adhesive comprises a minority proportion of one or more silicon-containing components such as a silicon-containing polymer. An example of such a polymer is a silicone/acrylic copolymer. Adding a silicon-containing polymer to the adhesive formulation enhances the reduction in adhesion that is realized upon application of a silicone debonding fluid. Silicone/acrylic copolymers are advantageous because they can be dissolved in an acrylic pressure sensitive adhesive, whereas most silicone polymers would be immiscible and tend to form separate phases within the adhesive. The amount of silicon-containing polymer in the adhesive formulation generally ranges from 0.5% by weight to 10% by weight, and typically from 2% by weight to 5% by weight, based upon the total weight of the adhesive composition.

Representative, non-limiting examples of silicone/acrylic copolymers that are commercially available and which can be used in the adhesives described herein include silicon/acrylic copolymers available under the designation Dow Corning FA 4001 CM or FA 4002 ID (acrylic/ polytrimethylsiloxymethacrylate copolymers). Additional examples of suitable commercially available copolymers include those under the designation Shin-Etsu KP-541, KP-543, KP-545, KP-550, KP-575 (acrylic polymers grafted with polydimethylsiloxane side chains).

The pressure sensitive adhesive also comprises a majority proportion of one or more acrylic polymers. Non-limiting examples of acrylic pressure sensitive adhesive with which the silicone/acrylic copolymer is blended, include those available under the designation Avery Dennison 1807, 1832, or S2510.

In particular versions, the silicone/acrylate copolymer which is incorporated in the pressure sensitive adhesive, is commercially available from Grant Industries under the designation GRANACRYSIL BMAS. The copolymer is utilized at a weight percentage of 5%, based upon the total weight of the adhesive. Specifically, this copolymer is a grafted silicone compound that is an isobutyl methacrylate/bis-hydroxypropyl dimethicone acrylate copolymer.

GRANACRYSIL BMAS is a 40% solution of a copolymer of isobutylmethacrylate and silicone-grafted acrylate in isoparaffin. This solution dries quickly on the skin to form an excellent clear, glossy film that is flexible and breathable. The film exhibits outstanding adhesion and water-repellency and can be readily removed and/or debonded by the exposure to debonding agents such as cyclomethicone, alcohol or mineral spirits. The solution is compatible with a variety of materials including cyclomethicone, dimethicone, hydrocarbon fluids, mineral oils, esters and fatty oils/waxes. GRANACRYSIL BMAS is an ideal film-former. Specifically, the copolymer of GRANACRYSIL BMAS is isododecane (and) isobutylmethacrylate/bis-hydroxypropyl dimethicone acrylate copolymer.

Table 1 set forth below lists the physical properties of GRANACRYSIL BMAS.

Table 1 - Physical Properties of GRANACRYSIL BMAS

| Appearance | Clear liquid |
| --- | --- |
| Viscosity at Amorphous State (Brookfield DV-I+, Spindle LV3, 5.0 RPM) (Viscosity measured at 20°C) | 0.5 - 1.3 Pa·s (500 - 1,300 cPs) |
| Non-Volatile Matter, wt% (105°C, 1.5g, 3hrs, Gravity Oven) | 40.0-45.0 |
| Residual Monomer (Isobutyl Methacrylate) | <20 ppm |
| Preservatives | None |
| Shelf Life | Three years in a closed container |
| Packaging | 32kg drum |

Articles

1. Substrate, Backing or Facestock Layer

The substrate or facestock layer can be comprised of paper, polymer film, fibrous materials, fabric(s), or combinations thereof. A preferred material for use as the substrate or facestock layer is a nonwoven material. Paper nonwoven materials can be used. Medical grade nonwoven materials are well known in the art and commercially available from a wide array of suppliers. In certain versions, the substrate is a polymeric nonwoven material such as a polypropylene/polyethylene nonwoven material. The substrate or facestock layer can be coated selectively with a print binder material such as polyvinyl alcohol to assist or promote adhesion of the toner or ink to the surface of the sheet. In addition, the entire assembly can be coated or provided with an antimicrobial material to reduce potential contamination.

In certain versions of the present subject matter, the adhesives comprising a blend of an acrylic adhesive and at least one silicone/acrylate copolymer dispersed therein, are used in conjunction with a nonporous film.

The polymer resin can be chosen from a large variety of polymer resins. However, it should possess film forming properties, i.e., the polymer resin can be formed into a non-porous continuous film or a sheet using conventional techniques known in the art. The polymer resin may or may not be hydrophilic in nature depending on the characteristics and/or properties desired for a particular end use. For example, the more hydrophilic the polymer resin, the higher would be the water vapor transmission rate (WVTR) through a non-porous film of the polymer resin. A convenient measure of the hydrophilic nature of a polymer resin is the amount of water absorbed by the unit weight of the polymer resin under specified conditions. ASTM D570 is such a standard test method for determination of water absorption of polymeric materials.

Other than film forming properties, the polymer resin can be derived from a wide variety of classes of polymers. The polymer resin can be either thermoplastic or thermosetting in nature. It is also possible that instead of a single polymer resin, a blend of two or more polymer resins is used. Some typical examples of preferred polymer resins are those made from poly(urethanes), poly(esters), poly(amides), copolyethersteramides, copolyesters, copolyetheresters, as homopolymers or as copolymers or as mixtures.

The polymer resin useful herein include block copolymers of either the polyurethane family or the copolyetheresteramide family or the copolyetherester family. The copolyetheresteramide polymers are a part of the general family of polyether block amide polymer chemistry as discussed in U.S. patent 4,230,838 to Foy et al. which also discusses the potential for formulating hydrophilic grades using poly(alkyleneoxide) glycol for the polyether block.

The preferred copolyester polymers are segmented copolyesters that contain recurring long chain ester units of a dicarboxylic acid and a long chain glycol, and also contains recurring short chain ester units of a dicarboxylic acid and a short chain glycol. The glycol can contain ether units, in which case the copolyester is sometimes referred to as a copolyetherester. The development of these polymers is described in U.S. Patent 4,493,870 to Vrouemraeto and U.S. Patent 4,725,481 to Ostapchenko describes compositions for hydrophilic copolyetherester polymers.

The preferred polyurethane polymers can be in different physical form, characterized principally on the basis of processing considerations. Preferably, the polyurethane polymers are either high molecular weight thermoplastics or relatively lower molecular weight prepolymers that are subsequently crosslinked to obtain the final polymer. These polyurethane polymers are segmented block copolymers based on a wide variety of precursors and are obtained by using different chemical routes.

It is well known in the art (see for instance, Hepbum, C., Polyurethane Elastomers, Applied Science Publishing Ltd., 1982 and Saunders S. Frisch, Polyurethanes chemistry and Technology, Part II Technology, Robert E. Krieger Publishing Company, 1983) that an isocyanate, a polyol and a chain extender are the building blocks generally used to make polyurethane polymers. Depending on the equivalency ratios employed using these building blocks, various characteristics are achieved that dictate both the processing techniques to be employed to achieve the final desired polymeric product, and the properties of the final polymer.

For example, a high molecular weight thermoplastic polyurethane is obtained when the equivalency ratio of the isocyanate species to that of the active hydrogen species (polyol and chain extender) is close to unity. Thus, thermoplastic polyurethanes are substantially centered around 50% equivalents of isocyanate. The thermoplastic route to a solid polyurethane can be subdivided into two categories: those completely soluble in certain solvents and containing no chemical crosslinks before and after processing, and those materials possessing no initial cross-links, but which form a lightly crosslinked structure after a heated post-cure. The former class is more predominantly encountered and is commonly made by the reaction of essentially equivalents of isocyanate and active hydrogen functionality or a slight excess of the active hydrogen component. Products from this class have the drawback in that they are inherently sensitive to particular solvents and will swell extensively in some solvents and dissolve in others. This limits their application in some areas of application such as coatings, adhesives and sealants.

In the latter class of thermoplastic polyurethanes, the synthesis is similar; however, a slight excess of isocyanate is employed to generate a final polymer having a small amount of unreacted isocyanate groups. These isocyanate groups are then available for cross-linking the final polymer through allophonate and biuret formation. The cross-link density is low by this method and the final thermoplastic polyurethane polymer must be given a heated post-cure to "activate" these residual "dormant" isocyanate groups.

The processing of either class of thermoplastic polyurethanes may proceed by melt processing techniques, as well as, by solution processing techniques. Melt processing, such as injection molding, extrusion, calendaring and film blowing are done usually at high temperatures. Solution processing usually involves solvating the thermoplastic polyurethane in very polar solvents such as tetrahydrofuran, dimethylformamide, dimethylsulfoxide, n-methyl pyrrolidone before using the polymer solution in processes such as coating, dipping, molding and spraying.

Polyurethane prepolymers, also useful in the present subject matter, are obtained by employing an equivalency ratio of isocyanate species to the active hydrogen species which is considerably greater than one such that the prepolymer has unreacted isocyanate groups. The curing of these prepolymers can be achieved by different techniques. In a "two-package" system (see U.S. Patent 3,471,445 for example) the prepolymer is mixed with a chain extender. The reacting mixture is then fabricated into its final form by an appropriate technique such as spraying, coating, casting or molding, and complete curing is then achieved by a post-cure at temperatures in excess of 100°C for 3 to 24 hours. In a "one-package" method (see U.S: Patent 4,532,316 for example), the curing is achieved by chain extension of the prepolymer through the reaction of the isocyanate groups with water or ambient moisture and cross-linking by allophanate and biuret formation. In another "one-package" system (see U.S. Patent 5,209,969 for example), curing of the prepolymer is achieved by using a "blocked" isocyanate, in which a polyol is employed as a mixture of a polyisocyanate (block isocyanate) masked with a blocking agent. The mixture is storage stable at under normal ambient conditions, but upon heating to an elevated temperature the isocyanate groups are unblocked thereby allowing the groups to react with the available hydrogen species provided by the polyol and/or the chain extender.

The physical form of these polyurethane prepolymers ranges generally from a viscous liquid to a waxy, low melting solid, depending usually upon whether a polyether or polyester polyol has been employed, respectively. In general, polyether prepolymer systems do not exhibit any of their final physical properties until substantially along in the cure cycle. Many polyester prepolymer systems, due to inherent tendency of the polyester segment to crystallize, exhibit many of their final physical properties early in the cure cycle. The physical form of the polyurethane prepolymers allows these to be processed by conventional liquid/hot melt processing techniques such as coating, spraying, casting, dipping and molding without the need for using any organic solvents.

The polyurethane polymers useful in the present subject matter can vary in the degree of their hydrophilic properties. It has been found in general, however, that for polyurethane polymers at the same amount of absorbent particle in the polymer composition, the percent increase in MVTR is larger as the hydrophilicity of the polymer resin decreases. Typically, hydrophilic characteristics of a polyurethane can be improved by introducing increasing amounts of oxyethylene units through the use of a polyol of polyoxyethylene as described in U.S. Patent 4,194,041 to Gore and U.S. Patent 4,532,316 to Henn as also in publications like those on page 1419 of volume 47 of Journal of Applied Polymer Science by N. S. Schneider, J. L. Illinger and F. E. Karansz. Hydrophilic polyurethane polymers are also commercially available in various forms - as solid resin, as a reactive prepolymer and as a solution in organic solvent or solvent mixtures.

It will be understood that the present subject matter includes the use of a wide array of nonporous films.

2. Flow Control Layer

A significant feature of the present subject matter is the provision of an interior flow control layer in the sheeted articles described herein. That layer defines a plurality of conduits, apertures, perforations, slits, or other means that enable controlled passage of one or more agent(s), such as an adhesive debonding agent, from one face of the layer through the thickness of the layer, to the other oppositely directed face of the layer. After having passed through the layer and to its other face, the agent(s) can then contact the adherend directly or travel further through the interface and/or laminate as desired.

In accordance with the present subject matter, many embodiments of the interior flow control layer exhibit a controllable flow profile across the thickness of the interior layer. The term "controllable flow profile" as used herein refers to the arrangement, location, shape and configuration of the passageways or conduits extending through the interior layer. Preferably, although not necessarily, the shape and configuration of each passageway is maintained relatively constant across the thickness of the interior layer. This aspect provides significantly greater ability to control the transport characteristics of the agent from one face of the layer to another face of the layer. Furthermore, this feature is readily distinguishable from prior art materials such as porous paper or fibrous layers in which void regions may extend from one face of the material to another face. In those materials, such intrinsic interior voids exhibit a great range of lengths, interior surface area, shapes, and configurations, all of which effect agent transport. Such widely varying voids makes controlling transport characteristics through the material suboptimal and seldom provide the desired control.

The size of the conduits or apertures defined in the interior flow control layer of the sheeted articles may range from 0.0127 mm (0.5 mils) to 50.8 mm (2000 mils), preferably from 0.0254 mm (1 mil) to 10.16 mm (400 mils), and more preferably from 0.254 mm (10 mils) to 7.62 mm (300 mils). These dimensions of aperture size are average diameters for circular shaped apertures that potentially afford uniform fluid egress. For non-circular shaped apertures, these values represent effective diameters. It will be appreciated that the present subject matter includes sizes greater than or less than these sizes.

Furthermore, the conduits or apertures may all be of the same size or of different sizes. Depending upon the particular application and/or laminate structure, it may be desirable to form a collection of apertures of one size in particular location(s) in the interior layer, and form a collection of apertures of another size in other location(s) in the interior layer. Moreover, it is contemplated that only one or more portions of the interior layer may define apertures, and other portions be free of apertures. In this regard, it may be beneficial to define a collection of apertures in only a central portion of an interior layer and leave the remaining regions of the layer aperture free or vice versa.

The conduits or apertures may be in the form of nearly any shape, such as circular, square, rectangular, triangular, poly-sided, irregular, slit-shaped, etc. Again, the particular selection of aperture shape(s) or combination of shapes will depend upon the particular application and/or laminate structure. Alternatively, conduits may also comprise unique materials that selectively afford ready transport to matched fluidic agents.

The number of conduits or apertures defined in the interior flow control layer may also vary. However, a typical number may be from 0.775 to 77.5 per $cm^2$ (5 to 500 per square inch), preferably from 1.55 to 38.75 per $cm^2$ (10 to 250 per square inch), and more preferably from 3.1 to 31 per $cm^2$ (20 to 200 per square inch ($in^2$)) of the flow control layer. It will be understood that the present subject matter includes the use of a greater or lesser number. It is also contemplated that the density of apertures, i.e. the number of apertures per unit of area of the layer, may vary at different locations along the layer. For example, it may be preferred for certain applications to provide for a relatively high aperture density within a particular region of the layer, and a lower aperture density in other regions. The present subject matter includes varying aperture density.

The selection of the size, shape, number of apertures, and aperture density defined in the interior layer determine the percentage or proportion of the surface area of the interior layer that permits passage of debonding agent(s) therethrough. Generally, for many applications, the percentage of apertured surface area of the interior flow control layer is at least 10% and typically from 10% to 90%, preferably 15% to 85%, and most preferably from 20% to 80%. It will be understood that the present subject matter includes laminates utilizing interior layers having percentage openings greater than or lesser than these amounts. Furthermore, it is to be understood that the present subject matter includes interior layers having different aperture percentages along different regions of the layer.

The controllable flow profile characteristic(s) of the flow control layer is such that the flow control layer may exhibit any one or more of the previously described average conduit or aperture diameters, number of conduits or apertures, and/or the percentage of apertured surface area. Moreover, the flow control layer used in the articles described herein may exhibit other characteristics in addition to, or instead of, the characteristics noted herein.

In certain versions of the present subject matter, the flow control layer includes, or is provided in the form of, a non-adhesive grid. Specifically, a grid of non-adhesive lines or outwardly extending ridges or projections is applied to the surface of the adhesive to enable the rapid ingress of the debonding fluid to the adhesive interface. The non-adhesive pattern can be created by flexographically printing a UV-cured ink onto a release liner. The ink is formulated such that the ink can wet out enough to print uniformly, but once cured it can be easily peeled away from the release liner's surface. An adhesive brought into contact with the printed release liner can be peeled off, taking the non-adhesive grid with it.

Adhesive can be applied to the printed liner in at least two different techniques. In one technique, adhesive can be applied by directly coating and drying an adhesive solution on the printed liner. In another technique, adhesive can be applied by laminating a dry adhesive film onto the printed liner.

In a particular version of the subject matter, a UV curable ink is used which is commercially available from Sericol under the designation PFS37169.

3. Carrier Sheet or Release Liner

In all of the noted embodiments, the carrier sheet can be comprised of paper, polymer film, or combinations thereof. The carrier sheet may be transparent to permit visibility of the facestock layer through the carrier sheet, as well as through other layers between the carrier sheet and the facestock layer. Thus, the use of transparent polymer films as the carrier sheet is contemplated. In other embodiments, the carrier sheet may be opaque or comprise one or more coloring agents such as whitening agents or pigments. Translucent carrier sheets are also contemplated. The outer surface or underside of the carrier sheet may have a release coating adhered to it to facilitate stacking of the products. Any release coating known in the art can be used. Silicone release coatings are especially useful. Untreated polyester film can be used. The carrier sheet typically has a thickness of from 6.4 microns (0.25 mils) to 2.5 microns (0.1 mils), and in one embodiment 13 microns (0.5 mils) to 125 microns (5 mils), and in one embodiment 50 microns (2 mils).

4. Adhesive Overlay Strip

As described in greater detail herein, one or more adhesive overlay strips may be disposed in a peel zone provided on the sheet article.

The material for the adhesive overlay strip, if used, can be comprised of paper, polymer film, fibrous materials, or combinations thereof. Preferably, the overlay material is formed from the same material as the substrate or facestock layer, and thus is preferably a nonwoven material.

Debonding Agent

The present subject matter contemplates the selective administration of one or more adhesive debonding agents into the multilayer articles described herein. In certain versions, the agent(s) are introduced into the laminates by exposing the apertured interior layer of the laminate and administering the desired agent(s) onto a top face of that layer. Agents that can be beneficially introduced, on demand, into the article may include, but are not restricted to, beneficial additives such as anesthetics, analgesics, and cooling/heating agents, etc. Although a wide array of agents can be introduced into the laminates described herein, the agent(s) include at least one adhesive deactivating or debonding agent. Silicone or perfluoroalkyl derivatives are particularly effective in deactivating skin adhesives. Various adhesive deactivating agents are described in greater detail herein.

The application of the agent(s) can be made mess-free by delivering them through (i) secondary carrier devices such as by a spray, roll-on or brush-on container, (ii) individually sterile packed "wet wipe" dispensers, (iii) "skin barrier" like applications that deliver the agent especially from below and "skin over" applications for optimum retention, (iv) impregnated gauze/foam carriers, and/or (v) encapsulated "release-on-demand" mediums to precisely meter or measure out needed amounts. It is also contemplated to utilize encapsulated microcapsules or microshells. The shells rupture if the laminate is stretched or subjected to any sort of trauma releasing the adhesive deactivating agent allowing for easy removal of the laminate. The application of the agent(s) can also be accomplished by delivering them through channels incorporated in the adhesive layer of the multilayer article which allows for rapid ingress of an adhesive deactivating agent thus allowing for easy removal of the article. Many embodiments of the delivery system and methods may be engineered into the laminate system, e.g. the top cover layer may additionally carry encapsulated agents on the outside so that once it is peeled away, it can be reapplied while presenting the opposing face such and appropriated stimulated to induce delivery of the agent into the conduits contained within the inner laminate layers. One or more of these techniques allows for controlled mess-free sterile dispensation that can be tailored to work optimally for a given adhesive. Assuming that the dominant deactivating mechanism is interfacial bond fracturing, the total amount needed is expected to be quite meager and easy to handle.

The selection of one or more fluid agents is governed by considerations such as the agents being readily available, safe to use, diffuse/reach the skin interface efficiently, deactivate adhesion rapidly but not unduly compromise the adhesive and/or carrier film so as to cause cohesive failure to leave a mess, not unduly modify the skin surface so subsequent adhesion is less robust such as in leaving a low energy coating like silicone, and not cause allergic reactions or other adverse reactions.

The terms "adhesive deactivating agent" or "adhesive debonding agent" as used herein refer to any agent or combination of agents that serve to reduce and preferably eliminate an adhesive bond between an adhesive and a substrate, which as described herein is typically mammalian skin. The adhesive deactivating agent is typically in a fluid form and exhibits a viscosity at generally ambient conditions and other properties and characteristics such that the agent can travel through the various apertures in the multilayer laminates described herein and reach the adhesive and preferably at least a substantial portion of the adhesive interface.

An important class of compounds for use as the adhesive deactivating agent is silicones including methicones and dimethicone (also known as polydimethylsiloxane) derivatives such as Toray fluids available from Dow Chemical Corp., tetramethysilane, hexamethyldisiloxane (HMDS) and their higher homologues. As noted, the adhesive deactivating agent may also include one or more perfluoroalkyl derivatives. Additional classes of components for use in adhesive deactivating agents include, but are not limited to low molecular weight oils; water with soap, pH modifiers, and/or containing other modifiers and ingredients; beneficial esters such as isopropyl myristate, triglyceral caproates, tetrahydrofurfural acetate or other esters and alkyl esters; limonene derivatives; paraffinic solvents; hydrocarbon solvents; various alkyl ethers; aromatic esters, surfactants, agents typically used in facial/mascara remover chemistries; hair spray ingredients; dermal medicants/lotions; allergy/inflammation/anesthetic agents such as for example Dermaplast spray from Medtech and related agents; and combinations thereof. Additional examples of compounds suitable for use as the adhesive deactivating agent or for use in association with such agent are described herein.

Particularly preferred silicones or rather polysiloxanes include, but are not limited to, dimethyl silicones or dimethylpolysiloxanes having the general formula $(-CH_3)_2-SiO)_x$, cyclic or straight chain, where x is a number of 2 to several hundred, i.e. 300. Trimethylsiloxy end-blocking units may be used for stabilization.

Choice of the agent is particularly important as depicted in Table 2 below which lists the relative effects of various adhesive debonding agents upon measured peel adhesions. Specifically, samples were adhesively bonded to a high density polyethylene (HDPE) substrate and allowed to dry for three days. Then, an effective amount of an adhesive debonding agent as listed in Table 2 was applied. The 90° peel force was then measured.

Table 2: Effects of Various Adhesive Debonding Agents

| Samples | Avg. Peel [N/cm] (N/in) | 95% CL |
|---|---|---|
| Control | 0.981 (2.4925) | 0.11 |
| frizz gel | 0.425 (1.079) | 0.00 |
| frizz spray | 0.406 (1.0305) | 0.01 |
| Sephora demaquillant | 0.393 (0.999) | 0.04 |
| Tetrahydrofurfuryl acetate | 0.381 (0.969) | 0.04 |
| PEG400 | 0.367 (0.933) | 0.00 |
| Control w/ small holes | 0.340 (0.863) | 0.04 |
| Uni-Solve | 0.299 (0.76) | 0.06 |
| triacetin (glycerol triacetate) | 0.297 (0.7555) | 0.02 |
| FZ-3196 | 0.259 (0.6575) | 0.09 |
| Sephora effaceur de maquillage | 0.172 (0.4365) | 0.02 |
| HMDS | 0.161 (0.4085) | 0.02 |
| Niltac Wipes | 0.155 (0.3935) | 0.01 |
| Dow Corning 2-1184 | 0.128 (0.325) | 0.02 |
| Sephora Face | 0.040 (0.1025) | 0.02 |
| Lancome | 0.039 (0.098) | 0.02 |
| Niltac Spray | 0.028 (0.072) | 0.01 |

Regarding the various debonding agents listed in Table 2, most are self explanatory. The "frizz" products are commercially available hair care products. Sephora demaquillant is a makeup remover formulation available from Sephora USA, Inc. of San Francisco, California. PEG 400 is polyethylene glycol 400. Uni-Solve is available from Smith & Nephew. FZ-3196 is a volatile alkyl methyl siloxane fluid from Dow Corning. Sephora effaceur de maquillage is a commercially available composition for erasing makeup, from Sephora. HMDS is hexamethyldisilazine also known as bis(trimethylsilyl)amine. Dow Corning 2-1184 is a mixture of volatile linear polydimethylsiloxanes. Sephora Face is a formulation for completing makeup removal from Sephora. Lancome is a commercially available composition available under that designation.

It is contemplated that the present subject matter may also utilize one or more agents based upon chemistries that allow for coating hair, such as for example by coating with amino polydimethylsiloxane (PDMS) which may selectively adhere through quaternary amine salt formation with surface acid groups believed to be present from oxidation of cystine di-sulfides, or fluro esters, etc. Such low energy coatings could then help ease or eliminate hair pull induced pain during peeling off or otherwise removing the adhesive product.

Other embodiments in accordance with the present subject matter include chemistries that can be appropriately combined with "switchable adhesive" technologies, for example using heat and/or water to help debond as described in US Patents US 5,183,841 and 5,385,965.

Another example of an adhesive deactivating agent is a debonding agent of fugitive silicones. Additional adhesive deactivating agents come in many forms including but not limited to wipes and sprays that can be individually packed and/or provided in a sterile container. The adhesive deactivating agent can be applied in a variety of different ways and using a wide array of strategies and techniques. For example, the agent can be applied via a secondary carrier device. The agent can also be applied via a skin barrier-like application. The agent could also be applied via an impregnated gauze and/or foam carrier.

After administration of an effective amount of an adhesive deactivating agent onto an exposed face of the laminate, the agent travels or is otherwise transported to the vicinity of the adhesive interface along which bonding occurs between the laminate and the substrate such as a user's skin. Contact or exposure occurs between the deactivating agent and the adhesive which, as explained herein, results in a reduction or elimination of the previous adhesive bond. Because the amount of debonding agent necessary to result in debonding depends upon a multitude of factors including (i) the adhesive, (ii) the characteristics of the surface to which the adhesive is bonded, and (iii) environmental factors such as humidity and temperature, the amount is referred to herein as "an effective amount." In many instances application of from 1 ml to 10 ml of the debonding agent to the adhered strip will be sufficient to effect debonding of most adhesives. The amount of contact time between the agent and adhesive necessary to result in debonding depends upon a variety of factors namely relating to the composition and interaction between the agent and adhesive. However, it is contemplated that for many applications, sufficient contact may be from only several seconds up to several minutes. The present subject matter includes contact times shorter and longer than these representative times.

Not wishing to be bound to any particular theory, it is believed that delivering a deactivating fluid agent efficiently to the adherend-adhesive interface may well be a key factor that readily and rapidly compromises the interface. Several additional factors that may be important to maximize or further promote this desired outcome could include one or more of the following:

Molecular weight- small molecules are generally expected to exhibit higher levels of interfacial diffusion than larger ones.

Surface roughness of the adherend and adhesive surface- the rougher the interacting interface, the easier it may be for interfacial fluid ingress which is also expected to contribute towards lower initial peel adhesion levels.

Adhesive topology- voids, patterns and/or channels all will contribute to the efficiency of fluid delivery to the interface.

Adhesive chemistry- the nature of the adhesive and particularly with regard to its chemistry/solubility parameter(s), crosslinking, and phase structure are all important parameters.

Compatibility-the agent should be sufficiently compatible for favorable ingress through the laminate and to the interface, but not so compatible as to adversely compromise the adhesive bulk and cause cohesive failures that can potentially leave residue. Effective interaction with the substrate (e.g. skin chemistry, physiology and/or surface exudates) can be particularly important.

Pain Mitigation- mitigating the pain of removal by, perhaps, advantageously interacting with the skin surface to dissolve/weaken interfacial exudates or bonds, minimize irritation by suppressing release of histamines, coating hair to minimizing their pulling, changing the skin topology to allow for easier debonding, providing for a physiological or even psychological relief, such as for example through cooling, warming and or wetting sensations.

In certain applications, it may be preferred to utilize an adhesive deactivating agent which is a medicant or medicant-like anesthetic, analgesic, cooling and/or heating agent, or combinations thereof.

Additional examples of compounds suitable for use as the adhesive deactivating agent or for use in association with such agent are provided in US Patents 2,552,520; 3,998,654; 4,324,595; 4,867,981; 5,004,502; 5,336,207;

6,063,231; 6,436,227; 7,354,889; 7,396,976; and 7,612,248. It will be understood that the present subject matter includes the use of one or more of these adhesive deactivating agents so long as the agent is effective in reducing the debonding force of the stretch releasable adhesive, and is appropriate for the end use application.

Representative Embodiments

Table 3 set forth below illustrates a particular layered arrangement and combination of materials for certain versions of the present subject matter.

Table 3 - Representative Embodiment of Sheeted Medical Article

| Layer | Material | Thickness/Weight | Source | Product ID |
|---|---|---|---|---|
| Backing | Paper Non Woven | 45 g/m$^2$ | Ahlstrom | 47718X |
| PSA | As Described Herein | 25-60 g/m$^2$ | As Described Herein | As Described Herein |
| Non-Adhesive Grid | UV Cured Ink | | Sericol | PFS37169 |
| Release Liner | Siliconized Paper | | | |

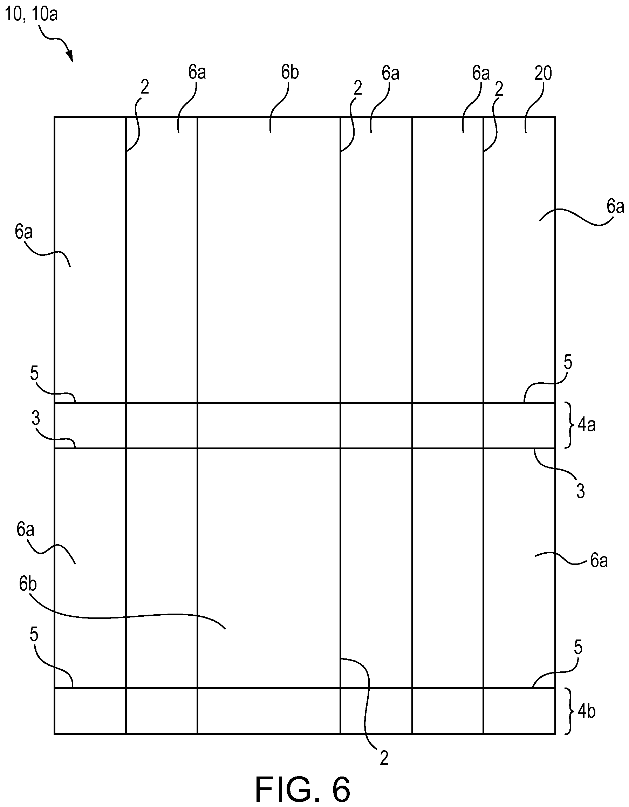

Figures 6-8 illustrate a representative embodiment of a sheeted medical article 10 in accordance with the present subject matter. The sheeted medical article 10 comprises a substrate or facestock layer 20, a flow control layer 30, one or more adhesive layers 40, and a carrier sheet 50. In many embodiments, the carrier sheet 50 includes a coating 52 of a release material as known in the art for contacting a face of the adhesive layer 40. As will be appreciated, the coating 52 facilitates or promotes separation of strips (described in greater detail herein) from the carrier sheet 50.

Typically, one or more longitudinal perforations or cut lines 2 are provided which extend lengthwise along the article 10 and typically parallel to one another and also parallel to at least one of the longitudinal edges of the article 10. The article 10 also typically includes one or more transverse perforations or cut lines 3 which extend across the article 10 and are typically transversely oriented to the longitudinal cut lines 2. In the article 10 depicted in Figures 6-8, a single transverse cut line 3 is provided. It will be understood that the articles of the present subject matter can utilize a wide range of combinations and arrangements of longitudinal and transverse cut lines, and quantities of longitudinal and transverse cut lines, and are in no way limited to the particular configuration depicted in the referenced figures.

The cut lines 2 and 3 define a plurality of adhesive strips shown as 6a and 6b in Figure 6. As a result of the arrangement of the cut lines 2 and 3, a first collection of strips 6a extend across a first region of the article 10 and a second collection of strips 6a extend across a second region of the article. The first and second regions are separated by the transverse cut line 3. In the particular embodiment shown, a single relatively wide strip 6b is provided in the first region, and another relatively wide strip 6b is provided in the second region of the article.

In certain versions of the articles, one or more non-adhesive regions or peel zones are provided typically along one or both ends of the adhesive strips 6a and 6b. The non-adhesive regions facilitate easy and convenient detachment of an adhesive strip 6a, 6b from the carrier sheet 50. Figure 6 depicts a first non-adhesive region 4a extending alongside and parallel to the transverse cut line 3, and a second non-adhesive region 4b extending alongside and parallel to an edge of the article 10. The non-adhesive regions 4a, 4b are free of adhesive and typically extend between a border line 5 and a transverse cut line such as cut line 3 to thereby define non-adhesive region 4a, or between a border line 5 and an edge of the article to thereby define non-adhesive region 4b.

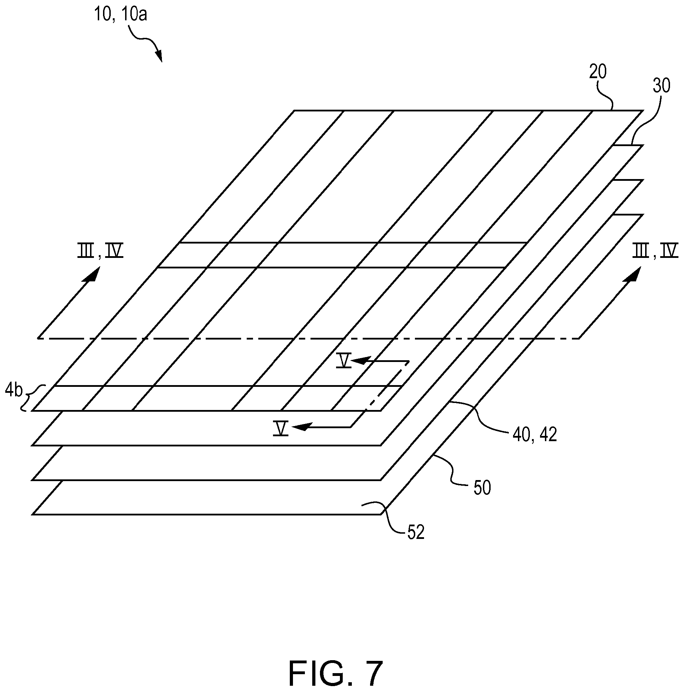

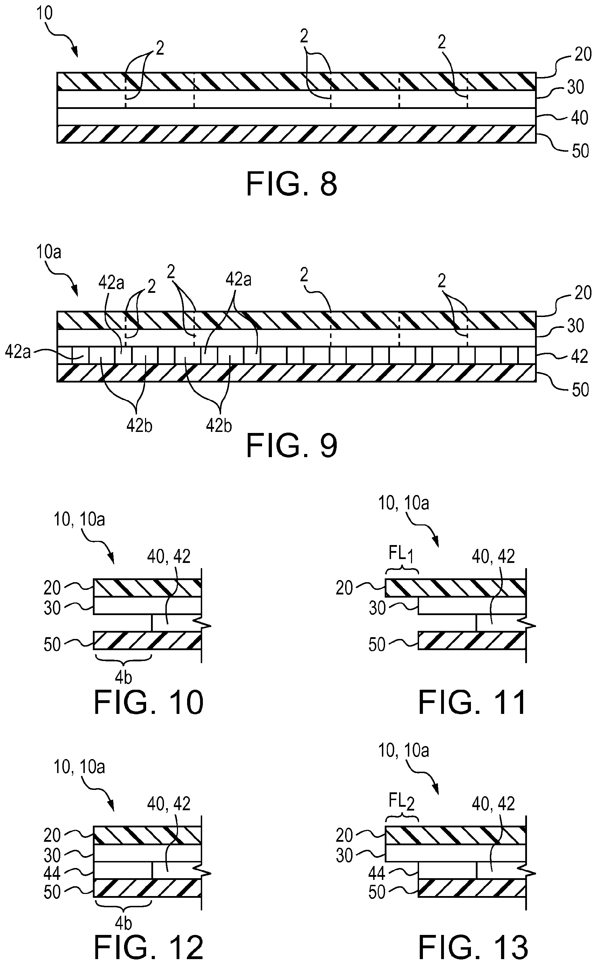

Figure 8 illustrates a schematic cross sectional view of the article 10 taken across line III-III in Figure 7. Figure 8 depicts the cut lines 2 and their provision through the substrate or facestock layer 20 and the flow control layer 30.

Figure 9 is a schematic cross sectional view of an alternative version of the article 10 of Figures 6-8, designated as article 10a. The article 10a utilizes the same configuration of layers as previously described and as shown in Figures 6 and 7. However, the article 10a includes an adhesive layer 42 instead of the previously described adhesive layer 40 that defines a plurality of flow passages or void regions 42a and a plurality of adhesive regions 42b. The flow passages or void regions 42a may be as previously described. It is also contemplated that the adhesive layer 42 can be formed or configured by adhesive pattern coating techniques.

Figure 10 is a schematic cross sectional view of an edge region of the articles 10, 10a taken across line V-V in Figure 7. As will be understood, Figure 10 illustrates the non-adhesive region 4b in greater detail. In one version of the articles 10, 10a, the non-adhesive region 4b is formed by undertaking measures so that the adhesive layer 40 or 42 does not extend to a distal edge of the article 10, 10a as shown in Figure 10. Alternatively, the non-adhesive region 4b is formed by providing one or more adhesive overlay strips 44 along the distal edge of the article within the non-adhesive region 4b as shown in Figure 12.

The articles 10 and 10a and various versions thereof may also include one or more finger-lift provisions along one or more distal edges of the articles. The finger-lift provisions may be provided in various forms, but can be readily provided by the use of extensions or regions of the substrate layer and/or accompanied by the flow control layer which extend beyond a distal edge of the carrier sheet. Specifically, in certain embodiments, the finger-lift provisions are in the form of an edge portion of the substrate layer which extends beyond a corresponding edge portion of the carrier sheet.

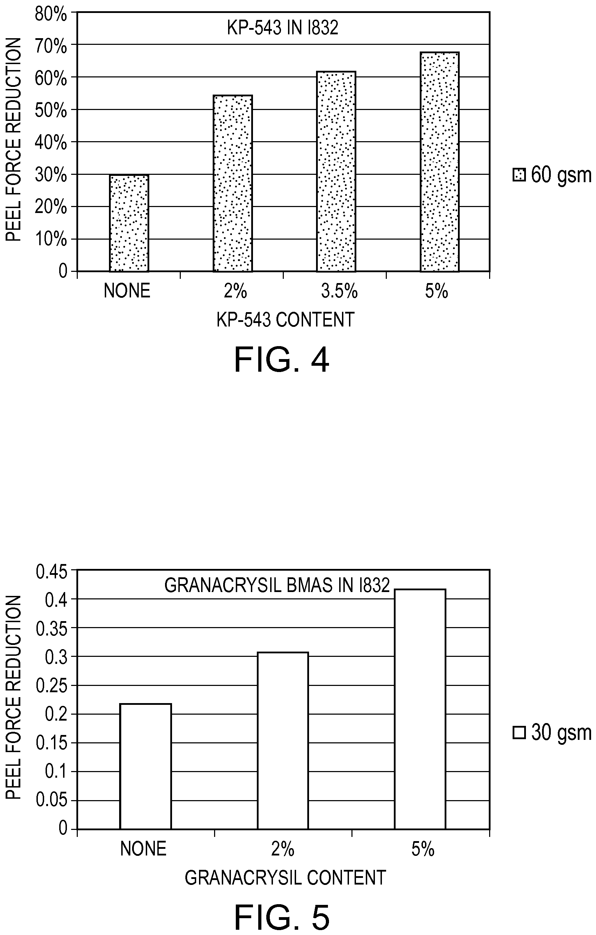

Figures 11 and 13 schematically depict two representative versions of finger-lifts, depicted as $FL_1$ and $FL_2$, respectively. Specifically, Figure 11 is a schematic cross sectional view similar to that of Figure 5, having a distal edge region of the substrate 20 extending beyond the distal edges of the flow control layer 30 and the carrier sheet 50. The version shown in Figure 11 features a region free of adhesive to provide the non-adhesive region. Figure 13 illustrates another finger-lift $FL_2$ in which both the substrate 20 and the flow control layer 30 extend beyond the distal edge of the carrier sheet 50. Figure 13 also depicts the combination of the finger-lift $FL_2$ and the adhesive overlay strip 44.

It will be appreciated that the present subject matter includes layered arrangements of materials and components different than the embodiments of Figures 6-14. For example, referring to Figure 7, the subject matter includes a multilayered assembly in which the adhesive layer 40 and the flow control layer 30 are replaced with one another. Thus, the adhesive layer 40 would be disposed immediately adjacent to the substrate or the facestock layer 20, and the flow control layer 30 would be disposed immediately adjacent to the carrier sheet 50.

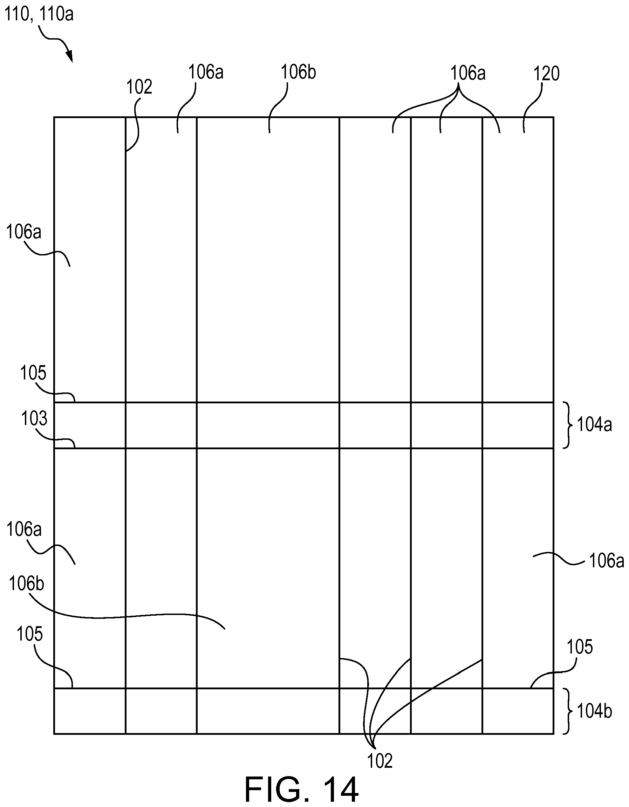

Figures 14-16 illustrate another representative embodiment of a sheeted medical article 110 in accordance with the present subject matter. The sheeted medical article 110 comprises a substrate or facestock layer 120, a flow control layer 130, one or more adhesive layers 140, 140a, and/or 140b, and a carrier sheet 150. In many embodiments, the carrier sheet 150 includes a coating 152 of a release material as known in the art for contacting a face of the adhesive layer 140. As will be appreciated, the coating 152 facilitates or promotes separation of strips (described in greater detail herein) from the carrier sheet 150. Specifically, in this embodiment, the article 110 utilizes two adhesive layers, a first adhesive layer 140a and a second adhesive layer 140b. The adhesive layers are separated by the flow control layer 130 disposed therebetween.

Typically, one or more longitudinal perforations or cut lines 102 are provided which extend lengthwise along the article 110 and typically parallel to one another and also parallel to at least one of the longitudinal edges of the article 110. The article 110 also typically includes one or more transverse perforations or cut lines 103 which extend across the article 110 and are typically transversely oriented to the longitudinal cut lines 102. In the article 110 depicted in Figures 14-16, a single transverse cut line 103 is provided. It will be understood that the articles of the present subject matter can utilize a wide range of combinations and arrangements of longitudinal and transverse cut lines, and quantities of longitudinal and transverse cut lines, and are in no way limited to the particular configuration depicted in the referenced figures.

The cut lines 102 and 103 define a plurality of adhesive strips shown as 106a and 106b in Figure 14. As a result of the arrangement of the cut lines 102 and 103, a first collection of strips 106a extend across a first region of the article 110 and a second collection of strips 106a extend across a second region of the article. The first and second regions are separated by the transverse cut line 103. In the particular embodiment shown, a single relatively wide strip 106b is provided in the first region, and another relatively wide strip 106b is provided in the second region of the article.

In certain versions of the articles, one or more non-adhesive regions or peel zones are provided typically along one or both ends of the adhesive strips 106a and 106b. The non-adhesive regions facilitate easy and convenient detachment of an adhesive strip 106a, 106b from the carrier sheet 150. Figure 14 depicts a first non-adhesive region 104a extending alongside and parallel to the transverse cut line 103, and a second non-adhesive region 104b extending alongside and parallel to an edge of the article 110. The non-adhesive regions 104a, 104b are free of adhesive and typically extend between a border line 105 and a transverse cut line such as cut line 103 to thereby define non-adhesive region 104a, or between a border line 105 and an edge of the article to thereby define non-adhesive region 104b.

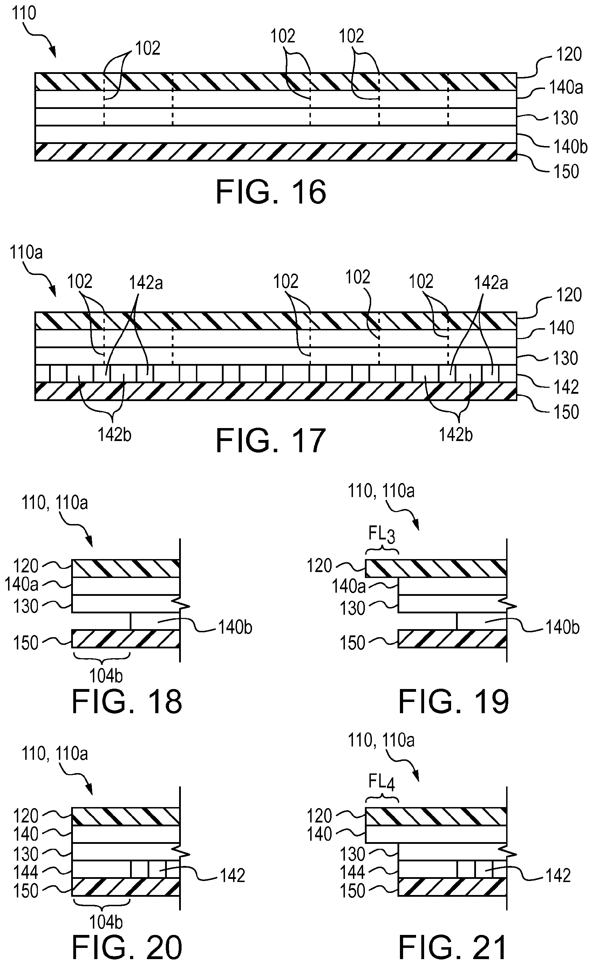

Figure 16 illustrates a schematic cross sectional view of the article 110 taken across line XI-XI in Figure 10. Figure 16 depicts the cut lines 102 and their provision through the substrate or facestock layer 120, a first adhesive layer 140a, and the flow control layer 130.

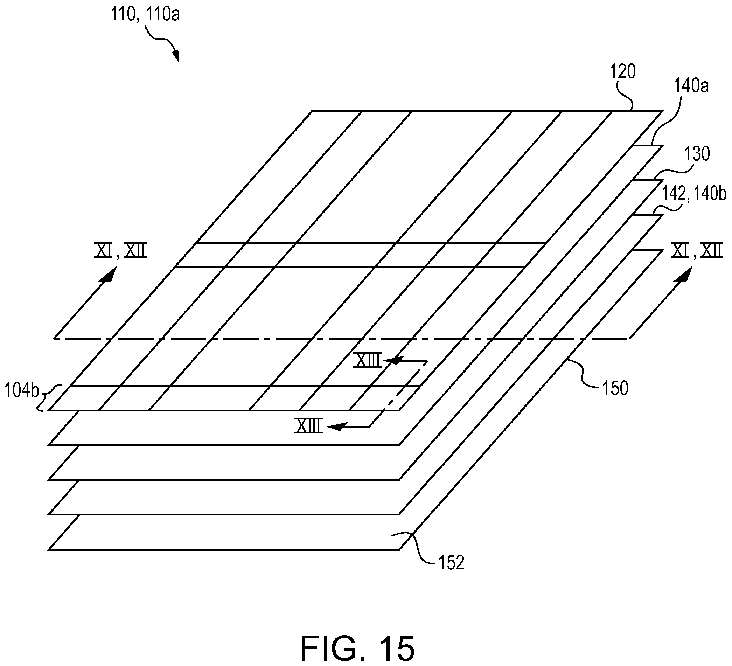

Figure 17 is a schematic cross sectional view of an alternative version of the article 110 of Figures 14-16, designated as article 110a. The article 110a utilizes the same configuration of layers as previously described and as shown in Figures 14 and 15. However, the article 110a includes an adhesive layer 142 instead of the second adhesive layer 140b in which the layer 142 defines a plurality of flow passages or void regions 142a and a plurality of adhesive regions 142b. The flow passages or void regions 142a may be as previously described. It is also contemplated that the adhesive layer 142 can be formed or configured by adhesive pattern coating techniques.

Figure 18 is a schematic cross sectional view of an edge region of the articles 110, 110a taken across line XIII-XIII in Figure 15. As will be understood, Figure 18 illustrates the non-adhesive region 104b in greater detail. In one version of the articles 110, 110a, the non-adhesive region 104b is formed by undertaking measures so that the adhesive layer 140b or 142 does not extend to a distal edge of the article 110, 110a as shown in Figure 18. Alternatively, in another version of the articles 110, 110a, the non-adhesive region 104b is formed by providing one or more adhesive overlay strips 144 along the distal edge of the article within the non-adhesive region 104b as shown in Figure 20.

The articles 110 and 110a and various versions thereof may also include one or more finger-lift provisions along one or more distal edges of the articles. The finger-lift provisions may be provided in various forms, but can be readily provided by the use of extensions or regions of the substrate layer and/or accompanied by the flow control layer which extend beyond a distal edge of the carrier sheet. Figures 19 and 21 schematically depict two representative versions of finger-lifts, depicted as $FL_3$ and $FL_4$, respectively. Specifically, Figure 19 is a schematic cross sectional view similar to that of Figure 18, having a distal edge region of the substrate 120 extending beyond the distal edges of the flow control layer 130 and the carrier sheet 150. The version shown in Figure 19 features a region free of adhesive to provide the non-adhesive region. Figure 21 illustrates another finger-lift $FL_4$ in which both the substrate 120 and the flow control layer 130 extend beyond the distal edge of the carrier sheet 150. Figure 21 also depicts the combination of the finger-lift $FL_4$ and the adhesive overlay strip 144.

It will be appreciated that in the embodiments depicted in Figures 18-21, any of the features and various layers can be combined. Thus, any of the adhesive layers 140, 140a, 140b, and 142, can be utilized in combination with a finger-lift feature or without a finger-lift feature, and in further combination with an adhesive overlay strip or without such a strip. Furthermore, as previously described in conjunction with Figures 6-14, the present subject matter includes variations in the positioning and order of the layers of the assemblies in Figures 15-21.

Kits

The present subject matter also provides kits or systems which include combinations of the previously described sheet articles and debonding agents. Typically, the debonding agent is provided in a container such as a fluid dispenser or may be impregnated within an absorbent article such as a sponge or porous wipe. The fluid dispenser, for example a bottle with a droplet dispenser or with provisions for administering the debonding agent as a mist or spray, may be packaged separately or in combination with the sheet article(s). Similarly, impregnated absorbent articles containing debonding agent and packaged in a sealed wrap such as a foil envelope, may be packaged in combination with the sheet article(s). Providing the debonding agent in combination with the sheet articles in many instances is preferred for reasons of convenience. The present subject matter also includes providing one or more sheets of the articles separately from debonding fluid or containing microcapsules with debonding fluid.

Additional Aspects

Additional details of sheeted healthcare articles are provided in PCT/US2011/55745 filed October 11, 2011 owned by the present assignee. Additional details of systems, methods and materials for delivery and debonding on demand are provided in WO 2010/129299 filed April 27, 2010 owned by the present assignee.

Although the present subject matter has been described in terms of a plurality of rectangular strips arranged and retained on a carrier sheet, it will be understood that the pre-cut or preshaped articles may be provided in a wide array of other shapes. For example, the articles may be circular, oval, arcuate, or have a number of sides greater than or less than four, or be square shaped. It is also contemplated that different materials could be used for the substrate or facestock layer such as by using a first material in one region of the sheet and a second material in another region of the sheet. This strategy may be described for applications in which labels are also provided in combination with adhesive strips on a single sheet. In such applications, it would not be necessary to provide a flow control layer in the label regions.

The adhesive compositions described herein can be used in association with a wide array of medical articles. Nonlimiting examples of such articles include wound dressings, surgical dressings, medical tapes, athletic tapes, surgical tapes, sensors, electrodes, ostomy appliances or related components such as sealing rings, catheters, connector fittings, catheter hubs, catheter adapters, fluid delivery tubes, electrical wires and cables, negative pressure wound therapy (NPWT) components, surgical drains, wound draining components, IV site dressings, prostheses, stoma pouches, buccal patches, transdermal patches, dentures, hairpieces, bandages, diapers, medical padding for example liposuction padding, hygiene pads, corn and callous pads, toe cushioning pads, and pads for protecting and cushioning tube sites such as tracheotomy tubes. The medical articles include one or more regions or surfaces to which the adhesive compositions of the present subject matter are applied. Forming a layer, coating, or other region of adhesive on an article enables the article to be adhered to a wide range of surfaces, including skin. It will be understood that the present subject matter is not limited to any of these articles. Instead, the subject matter includes the use of the adhesive compositions with other articles besides those noted herein.

Methods

The present subject matter also provides various methods and strategies for effectively using the multilayer articles described herein.

In certain applications, it may be desirable to print identifying information or decorative designs on the articles. Typically, the sheet-like articles can receive print or other markings from conventional copiers and printers. Toner, pigment or other like materials can be applied to the articles such as along an outer face of the substrate. For example in many healthcare settings, it may be desirable to print a patient's name or associated identification number or bar code on each of the strips or other members provided on the sheet.

The articles are used by selecting one or more strips or other components on a sheet, and then removing the selected strip(s) from the underlying carrier sheet. As previously explained, removal is facilitated by separating a distal end of a strip from the carrier sheet within a peel zone. As will be recalled, typically the peel zone is free of adhesive or includes an adhesive overlay strip which covers an underlying adhesive region, thereby precluding adhesion between the adhesive layer and the substrate layer and other layer(s).

After removal of the strip(s) from the carrier sheet, an adhesive face is exposed along the underside of the strip(s). One or more surfaces of interest are identified, such as human skin. The strip(s) are then contacted with the surface(s) of interest and the strip(s) are adhered thereto.

The adhered strip(s) can be easily and painlessly removed by exposing the strip(s) to an effective amount of one or more debonding agents. Such exposing may be performed by administration or application of the debonding agent to the strip(s). Exposing may also be performed by release of debonding agent if carried by or incorporated within the strip(s). Upon such exposure, at least a portion of the debonding agent is transported to one or more regions along the adhered face of the strip to facilitate debonding of the strip.

Incorporating and/or utilizing a silicon-containing polymer in a pressure sensitive adhesive has several advantages for fluid-activated debonding. The adhesive becomes more responsive to silicone-based adhesive deactivating fluids, meaning that adhesion decreases more dramatically in the fluid's presence in comparison to a non-silicon-containing formulation, typically greater than 75% reduction, as compared to approximately 35%, respectively.

The adhesive remains compatible with silicone-based release liners. The silicon content is typically not high enough to cause liner lockup which would necessitate expensive release systems, e.g. fluorosilicone or Teflon.

Additional benefits of the present subject matter are as follows. The adhesive does not dissolve in the presence of silicone-based adhesive deactivating fluids, and therefore no sticky residue is left on the substrate after fluid-assisted removal. In addition, the adhesive remains relatively inexpensive since the silicon-containing polymer content is minimal (usually 5% or less). Preferably, the silicon-containing polymer is miscible with the majority component of the pressure sensitive adhesive, resulting in a transparent, homogeneous mixture. A typical formulation for example contains 2-10% of an acrylic/silicone copolymer mixed with 90-98% acrylic pressure sensitive adhesive.

The present subject matter is well suited for use in medical adhesive products where it is desirable to combine high adhesion during wear with low adhesion at the time of removal.

Furthermore, the present subject matter provides a cost-effective way to greatly enhance an acrylic adhesive's response to a deactivating fluid because the amount of silcone-containing polymer is minimal. Furthermore, this is a relatively straight forward modification to existing adhesive formulations.

Example 1

A series of investigations were performed to assess the characteristics of an adhesive comprising amounts of silicone/acrylate copolymers dispersed in an acrylic adhesive. Specifically, three silicone/acrylate copolymer additives were blended with acrylic adhesive solutions as follows.

Table 4 - Adhesive Compositions

| Sample | Description |
|---|---|
| A | KP-543<br>Supplier: Shin-Etsu Silicones<br>Acrylic/silicone copolymer supplied as 50% solids solution in butyl acetate |
| B | GRANACRYSIL BMAS<br>Supplier: Grant Industries<br>Copolymer of isobutylmethacrylate and silicone-grafted acrylate supplied as 40% solids solution in isoparaffin |
| C | FA 4002 ID<br>Supplier: Dow Corning<br>Acrylates/polytrimethylsiloxymethacrylate copolymer supplied as 40% solids solution in isododecane |

Procedure

In each of the following examples, adhesive blends were prepared according to the following procedure. Acrylic adhesive solution (typically 40% solids in a mixture of toluene, hexane, and ethyl acetate) was blended together with silicone/acrylate copolymer solution in the proportions that yield the desired composition on a dry weight basis. The acrylic adhesives used are commercially available from Avery Dennison under the designations 1807 and 1832. After thorough mixing the resulting solution was coated onto a release liner and dried in an oven to remove the solvent. The dried adhesive film was then laminated to a backing material to produce a pressure-sensitive adhesive tape.

Specimens of tape measuring 2.54 cm x 7.62 cm (1 inch x 3 inch) were applied to the surface of a non-biological skin model and allowed to dwell overnight. Removal was accomplished using a tensile tester to peel the specimens away from the test panels at a 90° angle and a rate of 100 mm/min. The force required to peel the tape was recorded throughout the removal process.

Approximately halfway through the removal, drops of hexamethyldisiloxane (HMDS) were applied to the peel front in sufficient quantity to keep it wet throughout the remainder of the peel.

Data analysis consisted of averaging the force profile during the beginning part of the peel (when HMDS was absent) and taking another separate average of the force profile during the final portion of the peel when HMDS was present. The percentage reduction in peel force was taken as the quantitative metric of de-bonding effectiveness as shown in equation (I):

$$Peel\ Force\ Reduction = \frac{F_{dry} - F_{wet}}{F_{dry}} \qquad (I)$$

Results

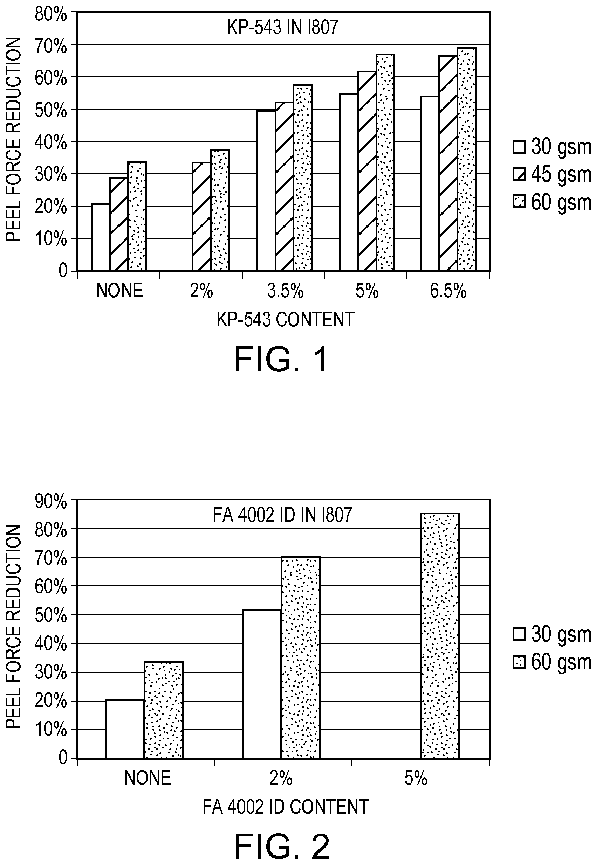

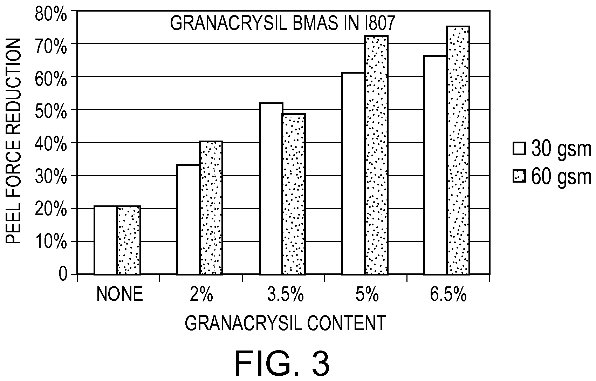

*Additives Blended with Avery Dennison Acrylic Adhesive I807:* Silicone/acrylate copolymers were blended with 1807 in various proportions up to 6.5% on a dry weight basis. Tapes were constructed using various coatweights of the adhesive blend: 30 g/m$^2$, 45 g/m$^2$, or 60 g/m$^2$. The measured peel force reductions from each tape are as follows, plotted together with unmodified I807 as a reference. These are shown in Figures 1-3.

*Additives Blended with Avery Dennison Acrylic Adhesive I832:* Silicone/acrylate copolymers were blended with 1832 in various proportions up to 5% on a dry weight basis. Tapes were constructed using various coatweights of the adhesive blend: 30 g/m$^2$ or 60 g/m$^2$. The measured peel force reductions from each tape are as follows, plotted together with unmodified 1832 as a reference. These are shown in Figures 4 and 5.

Measurements of Compatibility

Haze measurements were undertaken to assess the compatibility between the acrylic adhesive and the silicone/acrylate copolymer. Optical clarity is a good indication of compatibility, so a low haze value is indicative of a compatible blend. Mixtures of KP-543 with I-807 were found to have low haze values at all compositions studied, up to 6.5%. This indicates that KP-543 is soluble in the acrylic adhesive in these proportions. When blended with 1-807, GRANACRYSIL BMAS, on the other hand, became hazy when the concentration was increased above 3.5%. This indicates immiscibility of the two components above a threshold concentration.

Conclusions

In the evaluations described herein, combining silicone/acrylate copolymer with the acrylic adhesive improved the de-bonding response. Both acrylic adhesives when unmodified, exhibited peel force reductions less than 35%, but with the addition of silicone/acrylates, peel force reductions greater than 80% were achieved in some cases.

For most applications it will be important that the silicone/acrylate be soluble in the acrylic adhesive. Not all of the blends described herein exhibited good compatibility. KP-543 mixtures tended to yield better results than GRANACRYSIL BMAS when each was blended with I-807. The exact window of solubility can be established for any given combination of silicone/acrylate and adhesive.

Example 2

A collection of layered tape samples or specimens were prepared to further evaluate aspects of the present subject matter.

Specifically, a pressure sensitive adhesive composition comprising 5% by weight of a silicone acrylate copolymer available from Grant Industries under the designation GRANACRYSIL BMAS, was blended with 95% by weight of an acrylic pressure sensitive adhesive available from Avery Dennison Corporation under the designation 1807.

The mixture was coated on a siliconized polyethylene terephthalate (PET) release liner and then laminated to a paper non woven backing material and cut into 1 inch wide strips of tape. An equivalent construction was made using unadulterated acrylic pressure sensitive adhesive. The two constructions are summarized below in Table 5.

Table 5 - Tape Samples Tested

| Acrylic Tape | Silicone/Acrylic Tape |
|---|---|
| Paper Non Woven | Paper Non Woven |
| Acrylic PSA (30 g/m$^2$) | Acrylic PSA w/ Silicone Acrylate (30 g/m$^2$) |
| Siliconized PET Release Liner | Siliconized PET Release Liner |

Specimens of each tape were laminated to a non-biological skin model and left to dwell overnight before removing them using a peel tester. The first half of each specimen was peeled without the assistance of any deactivating fluid, and the second half was peeled while applying drops of HMDS to the peel front in sufficient quantity to keep the peel front wet for the remainder of the investigation. This method allows each specimen to function as its own internal standard when calculating the percentage reduction in peel force that occurs upon fluid application.

Both tapes' peel values without fluid were approximately 0.8 N/cm (2 N/in) (they were identical within the margin of error). When HMDS was applied to the peel front, the peel force of the acrylic pressure sensitive adhesive decreased 29% and the acrylic/silicone mixture experienced a 62% reduction. In neither case was there any evidence of residue left on the substrate after removal. Both tapes' siliconized release liners were easy to remove by hand.

To summarize this investigation, adding a mere 5% silicone acrylate copolymer to the adhesive caused its response to HMDS to more than double without sacrificing adhesive performance or causing liner lockup.

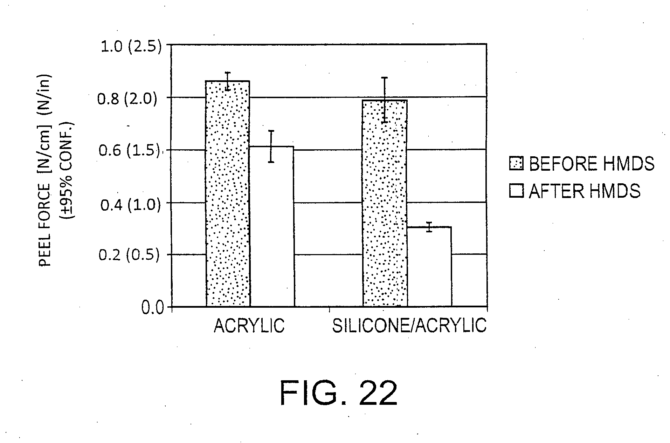

The results of peel testing of these samples are illustrated in Figure 22. 95% confidence intervals were calculated from four replicates.

Many other benefits will no doubt become apparent from future application and development of this technology.

As described hereinabove, the present subject matter overcomes many problems associated with previous strategies, systems and/or products. However, it will be appreciated that various changes in the details, materials and arrangements of components, which have been herein described and illustrated in order to explain the nature of the present subject matter, may be made by those skilled in the art without departing from the principle and scope of the claimed subject matter, as expressed in the appended claims.

**Claims**

1.  A multilayer article having a plurality of adhesive strips provided on a release liner, the strips adapted to be easily removed after application to a surface of interest by exposure to a debonding agent, the article comprising:

    a release liner defining a release face;
    a substrate layer having at least one cut line extending across its thickness and defining a plurality of adhesive strips;
    at least one adhesive layer extending between the release liner and the substrate layer, the adhesive layer including a pressure sensitive adhesive including (i) at least one silicone containing component, and (ii) at least one acrylic polymer;
    an interior flow control layer disposed between the release liner and the substrate layer, wherein the flow control layer defines a plurality of conduits or apertures from one face of the layer through the thickness of the layer to the other oppositely directed face of the layer so as to exhibit a controllable flow profile such that upon exposure to a debonding agent, the debonding agent is readily transported to the adhesive layer, wherein the debonding agent is an agent or combination of agents that serve to reduce and preferably eliminate an adhesive bond between the adhesive and the surface of interest.

**2.** The multilayer article of claim 1 wherein the pressure sensitive adhesive includes from 0.5% by weight to 10% by weight of the at least one silicone containing component, and from 90% by weight to 99.5% by weight of at least one acrylic component.

**3.** The multilayer article of any one of claims 1-2 wherein the at least one silicone containing component is a silicone/acrylic copolymer.

**4.** The multilayer article of claim 3 wherein the silicone/acrylic copolymer is an isobutyl methacrylate/bis-hydroxypropyl dimethicone acrylate copolymer.

**5.** The multilayer article of any one of claims 1-4 wherein the controllable flow profile of the flow control layer is such that the flow control layer defines a plurality of conduits or apertures having an average diameter of from 0.0127 mm (0.5 mils) to 50.8 mm (2000 mils), preferably from 0.0254 mm (1 mil) to 10.16 mm (400 mils), more preferably from 0.254 mm (10 mils) to 7.62 mm (300 mils).

**6.** The multilayer article of any one of claims 1-5 wherein the controllable flow profile of the flow control layer is such that the number of conduits or apertures defined in the flow control layer is from 0.775 to 77.5 per $cm^2$ (5 to 500 per square inch) of the flow control layer, preferably from 1.55 to 38.75 per $cm^2$ (10 to 250 per square inch) of the flow control layer, more preferably from 3.1 to 31 per $cm^2$ (20 to 200 per square inch) of the flow control layer.

**7.** The multilayer article of any one of claims 1-6 wherein the controllable flow profile of the flow control layer is such that the percentage of apertured surface area of the flow control layer is from 10% to 90%, preferably from 15% to 85%, more preferably from 20% to 80%.

**8.** The multilayer article of claims 1-7 wherein the flow control layer is a UV cured ink layer.

**9.** The multilayer article of any one of claims 1-8 wherein the at least one adhesive layer defines a plurality of voids or channels and a plurality of adhesive regions.

**10.** The multilayer article of any one of claims 1-9 wherein the at least one adhesive layer includes a first adhesive layer disposed alongside and in contact with the substrate layer and a second adhesive layer disposed alongside and in contact with the release liner.

**11.** The multilayer article of claim 10 wherein the flow control layer is disposed between the first adhesive layer and the second adhesive layer.

**12.** The multilayer article of any one of claims 1-11 wherein the article defines at least one peel zone.

**13.** The multilayer article of any one of claims 1-12 wherein the article includes at least one finger-lift such that at least an edge portion of the substrate layer extends beyond a corresponding edge portion of the release liner.

**14.** The multilayer article of any one of claims 1-13 wherein the substrate layer includes a nonporous film.

**15.** The multilayer article of claim 14 wherein the nonporous film is selected from the group consisting of poly(urethanes), poly(esters), poly(amides), copolyethersteramides, copolyesters, copolyetheresters, and combinations thereof.

**16.** The multilayer article of any one of claims 1-15 further comprising:
an effective amount of a debonding agent associated with the article wherein the debonding agent is incorporated in the article, or wherein the debonding agent is initially separate from the article and subsequently applied to the article.

**17.** A kit comprising:
the multilayer article of any one of claims 1-16; and (b) a debonding agent.

**18.** The kit of claim 17 wherein the debonding agent includes a silicone.

**19.** The kit of claim 18 wherein the silicone has the general formula $(-CH_3)_2 - SiO)_x$, wherein x is from 2 to 300.

20. The kit of any one of claims 17-19 further comprising:

(c) a container in which the debonding agent is stored prior to administration to the article.

21. The kit of any one of claims 17-20 wherein the kit is provided in a unitary package.

22. A method of facilitating removal of an article from a surface after the article is adhesively attached thereto, wherein the article includes a carrier sheet, at least one adhesive layer disposed along the carrier sheet, the adhesive layer including a pressure sensitive adhesive including (i) at least one silicone containing component, and (ii) at least one acrylic polymer, a substrate layer defining at least one cut line extending across its thickness, and a flow control layer disposed between the at least one adhesive layer and the substrate layer, the flow control layer exhibiting a controllable flow profile, the method comprising:
exposing the article to an effective amount of at least one debonding agent to thereby debond the adhesively attached article from the surface.

23. The method of claim 22 wherein exposing the article to an effective amount of at least one debonding agent is performed by administering the debonding agent to a region of the flow control layer whereby the debonding agent is transported to at least one other region of the flow control layer.

24. The method of claim 23 whereby the debonding agent is further transported to an adhesive interface defined between the at least one adhesive layer and the surface to which the article is adhesively attached.

25. A method for adhesively applying an article to skin and reducing discomfort associated with removal of the article from the skin, the method comprising:

providing an article including a carrier sheet, at least one adhesive layer disposed along the carrier sheet, the adhesive layer including a pressure sensitive adhesive including (i) at least one silicone containing component, and (ii) at least one acrylic polymer, a substrate layer defining at least one cut line extending across its thickness, and a flow control layer disposed between the at least one adhesive layer and the substrate layer, wherein the flow control layer exhibits a controllable flow profile;
adhesively applying the article to skin by contacting at least a portion of the pressure sensitive adhesive to the skin to thereby adhesively adhere the article to skin;
exposing the article to an effective amount of at least one debonding agent to thereby debond the adhesively attached article from the skin.

26. The method of claim 25 wherein exposing the article to an effective amount of at least one debonding agent is performed by administering the debonding agent to a region of the flow control layer whereby the debonding agent is transported to at least one other region of the flow control layer.

27. The method of claim 26 whereby the debonding agent is further transported to an adhesive interface defined between the at least one adhesive layer and the skin to which the article is adhesively attached.


**Patentansprüche**

1. Mehrschichtiger Gegenstand mit einer Mehrzahl von Klebestreifen, die auf einer Trennfolie bereitgestellt sind, wobei die Streifen derart beschaffen sind, dass sie nach dem Aufbringen auf einer Oberfläche von Interesse durch Exposition an ein Ablösemittel leicht entfernt werden können, wobei der Gegenstand umfasst:

eine Trennfolie, die eine Trennfläche definiert;
eine Substratschicht mit mindestens einer Schnittlinie, die sich über ihre Dicke erstreckt und eine Mehrzahl von Klebestreifen definiert;
mindestens eine Klebeschicht, die sich zwischen der Trennfolie und der Substratschicht erstreckt, wobei die Klebeschicht einen druckempfindlichen Klebstoff enthält, der (i) mindestens eine siliconhaltige Komponente und (ii) mindestens ein Acrylpolymer enthält;
eine innere Durchflussregelungsschicht, die zwischen der Trennfolie und der Substratschicht angeordnet ist, wobei die Durchflussregelungsschicht eine Mehrzahl von Kanälen oder Öffnungen von einer Seitenfläche der Schicht durch die Dicke der Schicht zu der anderen entgegengesetzt gerichteten Seitenfläche der Schicht definiert, um ein derartiges steuerbares Strömungsprofil aufzuweisen, dass bei Exposition an ein Ablösemittel, das Ablösemittel leicht zu der Klebeschicht transportiert wird, wobei das Ablösemittel ein Mittel oder eine Kombination von Mitteln ist, die dazu dienen, eine Klebeverbindung zwischen dem Kleber und der Oberfläche von Interesse zu reduzieren und vorzugsweise zu eliminieren.

2. Mehrschichtiger Gegenstand nach Anspruch 1, wobei der druckempfindliche Klebstoff von 0,5 Gew.-% bis 10 Gew.-% der mindestens einen siliconhaltigen Komponente und 90 Gew.-% bis 99,5 Gew.-% mindestens einer Acrylkomponente enthält.

3. Mehrschichtiger Gegenstand nach einem der Ansprüche 1-2, wobei die mindestens eine siliconhaltige Komponente ein Silicon/Acryl-Copolymer ist.

4. Mehrschichtiger Gegenstand nach Anspruch 3, wobei das Silicon/AcrylCopolymer ein Isobutylmethacrylat/Bis-hydroxypropyldimethiconacrylat-Copolymer ist.

5. Mehrschichtiger Gegenstand nach einem der Ansprüche 1-4, wobei das steuerbare Strömungsprofil der Durchflussregelungsschicht derart ist, dass die Durchflussregelungsschicht eine Mehrzahl von Kanälen oder Öffnungen mit einem durchschnittlichen Durchmesser von 0,0127 mm (0,5 mils) bis 50,8 mm (2000 mils), vorzugsweise von 0,0254 mm (1 mil) bis 10,16 mm (400 mils), bevorzugter von 0,254 mm (10 mils) bis 7,62 mm (300 mils), definiert.

6. Mehrschichtiger Gegenstand nach einem der Ansprüche 1-5, wobei das steuerbare Strömungsprofil der Durchflussregelungsschicht derart ist, dass die Anzahl der in der Durchflussregelungsschicht definierten Kanäle oder Öffnungen von 0,775 bis 77,5 pro cm$^2$ (5 bis 500 pro Quadratinch) der Durchflussregelungsschicht, vorzugsweise von 1,55 bis 38,75 pro cm$^2$ (10 bis 250 pro Quadratinch) der Durchflussregelungsschicht, bevorzugter von 3,1 bis 31 pro cm$^2$ (20 bis 200 pro Quadratinch) der Durchflussregelungsschicht, beträgt.

7. Mehrschichtiger Gegenstand nach einem der Ansprüche 1-6, wobei das steuerbare Strömungsprofil der Durchflussregelungsschicht derart ist, dass der prozentuale Anteil der mit Öffnungen versehenen Oberfläche der Durchflussregelungsschicht von 10% bis 90%, vorzugsweise 15% bis 85%, bevorzugter von 20% bis 80%, beträgt.

8. Mehrschichtiger Gegenstand nach den Ansprüchen 1-7, wobei die Durchflussregelungsschicht eine UV-gehärtete Farbschicht ist.

9. Mehrschichtiger Gegenstand nach einem der Ansprüche 1-8, wobei die mindestens eine Klebeschicht eine Mehrzahl von Hohlräumen oder Kanälen und eine Mehrzahl von Klebstoffbereichen definiert.

10. Mehrschichtiger Gegenstand nach einem der Ansprüche 1-9, wobei die mindestens eine Klebeschicht eine erste Klebeschicht, die neben und in Kontakt mit der Substratschicht angeordnet ist, und eine zweite Klebeschicht einschließt, die neben und in Kontakt mit der Trennfolie angeordnet ist.

11. Mehrschichtiger Gegenstand nach Anspruch 10, wobei die Durchflussregelungsschicht zwischen der ersten Klebeschicht und der zweiten Klebeschicht angeordnet ist.

12. Mehrschichtiger Gegenstand nach einem der Ansprüche 1-11, wobei der Gegenstand mindestens eine Abziehzone definiert.

13. Mehrschichtiger Gegenstand nach einem der Ansprüche 1-12, wobei der Gegenstand mindestens eine Abhebelasche einschließt, derart dass sich mindestens ein Kantenabschnitt der Substratschicht über einen entsprechenden Kantenabschnitt der Trennfolie hinaus erstreckt.

14. Mehrschichtiger Gegenstand nach einem der Ansprüche 1-13, wobei die Substratschicht einen nicht-porösen Film enthält.

15. Mehrschichtiger Gegenstand nach Anspruch 14, wobei der nicht-poröse Film ausgewählt ist aus der Gruppe, bestehend aus Poly(urethanen), Poly(estern), Poly(amiden), Copolyethersteramiden, Copolyestern, Copolyetherestern und Kombinationen davon.

16. Mehrschichtiger Gegenstand nach einem der Ansprüche 1-15, ferner umfassend:

19

eine wirksame Menge eines Ablösemittels, das mit dem Gegenstand verbunden ist, wobei das Ablösemittel in den Gegenstand eingebaut ist, oder wobei das Ablösemittel anfänglich von dem Gegenstand getrennt ist und anschließend auf den Gegenstand aufgebracht wird.

17. Kit, umfassend:
den mehrschichtigen Gegenstand nach einem der Ansprüche 1-16; und (b) ein Ablösemittel.

18. Kit nach Anspruch 17, wobei das Ablösemittel ein Silicon enthält.

19. Kit nach Anspruch 18, wobei das Silicon die allgemeine Formel $(-CH_3)_2 - SiO)_x$ aufweist, wobei x von 2 bis 300 ist.

20. Kit nach einem der Ansprüche 17-19, ferner umfassend:

(c) einen Behälter, in welchem das Ablösemittel vor der Verabreichung an den Gegenstand gelagert wird.

21. Kit nach einem der Ansprüche 17-20, wobei das Kit in einer einheitlichen Verpackung bereitgestellt ist.

22. Verfahren zum Erleichtern des Entfernens eines Gegenstands von einer Oberfläche nachdem der Gegenstand klebend daran angebracht ist, wobei der Gegenstand eine Trägerfolie, mindestens eine entlang der Trägerfolie angeordnete Klebeschicht, wobei die Klebeschicht einen druckempfindlichen Klebstoff enthält, der (i) mindestens eine siliconhaltige Komponente und (ii) mindestens ein Acrylpolymer enthält, eine Substratschicht, die mindestens eine Schnittlinie definiert, die sich über ihre Dicke erstreckt, und eine Durchflussregelungsschicht enthält, die zwischen der mindestens einen Klebeschicht und der Substratschicht angeordnet ist, wobei die Durchflussregelungsschicht ein steuerbares Strömungsprofil aufweist, wobei das Verfahren umfasst:
das Exponieren des Gegenstands einer wirksamen Menge von mindestens einem Ablösemittel, um dadurch den klebend befestigten Artikel von der Oberfläche zu lösen.

23. Verfahren nach Anspruch 22, wobei das Exponieren des Gegenstands einer wirksamen Menge von mindestens einem Ablösemittel durch Verabreichen des Ablösemittels an einen Bereich der Durchflussregelungsschicht durchgeführt wird, wodurch das Ablösemittel zu mindestens einem anderen Bereich der Durchflussregelungsschicht transportiert wird.

24. Verfahren nach Anspruch 23, wobei das Ablösemittel weiter zu einer Klebegrenzfläche transportiert wird, die zwischen der mindestens einen Klebeschicht und der Oberfläche, an der der Gegenstand klebend befestigt ist, definiert ist.

25. Verfahren zum klebenden Auftragen eines Gegenstands auf die Haut und zum Reduzieren von Beschwerden, die mit dem Entfernen des Gegenstands von der Haut verbunden sind, wobei das Verfahren umfasst:

das Bereitstellen eines Gegenstandes, enthaltend eine Trägerfolie, mindestens eine Klebeschicht, die entlang der Trägerfolie angeordnet ist, wobei die Klebeschicht einen druckempfindlichen Klebstoff enthält, der (i) mindestens eine siliconhaltige Komponente und (ii) mindestens ein Acrylpolymer enthält, eine Substratschicht, die mindestens eine Schnittlinie definiert, die sich über ihre Dicke erstreckt, und eine Durchflussregelungsschicht, die zwischen der mindestens einen Klebeschicht und der Substratschicht angeordnet ist, wobei die Durchflussregelungsschicht ein steuerbares Strömungsprofil aufweist;
das klebende Aufbringen des Gegenstands auf die Haut durch Inkontaktbringen von mindestens einem Teil des druckempfindlichen Klebstoffs mit der Haut, um dadurch den Gegenstand klebend an der Haut zu befestigen;
das Exponieren des Gegenstands einer wirksamen Menge von mindestens einem Ablösemittel, um dadurch den klebend befestigten Gegenstand von der Haut zu lösen.

26. Verfahren nach Anspruch 25, wobei das Exponieren des Gegenstands einer wirksamen Menge mindestens eines Ablösemittels durch Verabreichen des Ablösemittels an einen Bereich der Durchflussregelungsschicht durchgeführt wird, wodurch das Ablösemittel zu mindestens einem anderen Bereich der Durchflussregelungsschicht transportiert wird.

27. Verfahren nach Anspruch 26, wobei das Ablösemittel weiter zu einer Klebegrenzfläche transportiert wird, die zwischen der mindestens einen Klebeschicht und der Haut, an die der Gegenstand klebend befestigt ist, definiert ist.

**Revendications**

1. Article multicouche ayant une pluralité de bandes adhésives fournies sur un revêtement antiadhésif, les bandes étant adaptées pour être retirées facilement après application sur une surface d'intérêt par exposition à un agent de décollement, l'article comprenant :

   un revêtement antiadhésif définissant une face antiadhésive ;
   une couche de substrat ayant au moins une ligne de découpe se prolongeant à travers son épaisseur et définissant une pluralité de bandes adhésives ;
   au moins une couche adhésive se prolongeant entre le revêtement antiadhésif et la couche de substrat, la couche adhésive comprenant un adhésif sensible à la pression comprenant (i) au moins un composant contenant une silicone, et (ii) au moins un polymère acrylique ;
   une couche de régulation d'écoulement interne disposée entre le revêtement antiadhésif et la couche de substrat, dans lequel la couche de régulation d'écoulement définit une pluralité de conduites ou d'ouvertures à partir d'une face de la couche à travers l'épaisseur de la couche jusqu'à l'autre face opposée de la couche afin de présenter un profil d'écoulement régulable de façon que lors d'une exposition à un agent de décollement, l'agent de décollement soit facilement transporté jusqu'à la couche adhésive, dans lequel l'agent de décollement est un agent ou une combinaison d'agents qui sert à réduire et de préférence à éliminer une liaison adhésive entre l'adhésif et la surface d'intérêt.

2. Article multicouche selon la revendication 1 dans lequel l'adhésif sensible à la pression comprend de 0,5 % en poids à 10 % en poids de l'au moins un composant contenant une silicone, et de 90 % en poids à 99,5 % en poids de l'au moins un composant acrylique.

3. Article multicouche selon l'une quelconque des revendications 1 et 2 dans lequel l'au moins un composant contenant une silicone est un copolymère de silicone/acrylique.

4. Article multicouche selon la revendication 3 dans lequel le copolymère de silicone/acrylique est un copolymère de méthacrylate d'isobutyle/acrylate de bis-hydroxy-propyl diméthicone.

5. Article multicouche selon l'une quelconque des revendications 1 à 4 dans lequel le profil d'écoulement régulable de la couche de régulation d'écoulement est tel que la couche de régulation d'écoulement définit une pluralité de conduites ou d'ouvertures ayant un diamètre moyen de 0,0127 mm (0,5 mil) à 50,8 mm (2000 mils), de préférence de 0,0254 mm (1 mil) à 10,16 mm (400 mils), de manière davantage préférée de 0,254 mm (10 mils) à 7,62 mm (300 mils).

6. Article multicouche selon l'une quelconque des revendications 1 à 5 dans lequel le profil d'écoulement régulable de la couche de régulation d'écoulement est tel que le nombre de conduites ou d'ouvertures définies dans la couche de régulation d'écoulement est de 0,775 à 77,5 par cm$^2$ (5 à 500 par pouce carré) de la couche de régulation d'écoulement, de préférence de 1,55 à 38,75 par cm$^2$ (10 à 250 par pouce carré) de la couche de régulation d'écoulement, de manière davantage préférée de 3,1 à 31 par cm$^2$ (20 à 200 par pouce carré) de la couche de régulation d'écoulement.

7. Article multicouche selon l'une quelconque des revendications 1 à 6 dans lequel le profil d'écoulement régulable de la couche de régulation d'écoulement est tel que le pourcentage de surface ouverte de la couche de régulation d'écoulement est de 10 % à 90 %, de préférence de 15 % à 85 %, de manière davantage préférée de 20 % à 80 %.

8. Article multicouche selon l'une quelconque des revendications 1 à 7 dans lequel la couche de régulation d'écoulement est une couche d'encre durcie sous UV.

9. Article multicouche selon l'une quelconque des revendications 1 à 8 dans lequel l'au moins une couche adhésive définit une pluralité de vides ou de canaux et une pluralité de régions adhésives.

10. Article multicouche selon l'une quelconque des revendications 1 à 9 dans lequel l'au moins une couche adhésive comprend une première couche adhésive disposée le long et au contact de la couche de substrat et une seconde couche adhésive disposée le long et au contact du revêtement antiadhésif.

11. Article multicouche selon la revendication 10 dans lequel la couche de régulation d'écoulement est disposée entre la première couche adhésive et la seconde couche adhésive.

**12.** Article multicouche selon l'une quelconque des revendications 1 à 11 dans lequel l'article définit au moins une zone de pelage.

**13.** Article multicouche selon l'une quelconque des revendications 1 à 12 dans lequel l'article comprend au moins une tirette de sorte qu'au moins une partie de bord de la couche de substrat se prolonge au-delà d'une partie de bord correspondante du revêtement antiadhésif.

**14.** Article multicouche selon l'une quelconque des revendications 1 à 13 dans lequel la couche de substrat comprend un film non poreux.

**15.** Article multicouche selon la revendication 14 dans lequel le film non poreux est sélectionné dans le groupe constitué des poly(uréthanes), des poly(esters), des poly(amides), des copolyétherstéramides, des copolyesters, des copolyétheresters, et de leurs combinaisons.

**16.** Article multicouche selon l'une quelconque des revendications 1 à 15 comprenant en outre :
une quantité efficace d'un agent de décollement associé à l'article dans lequel l'agent de décollement est incorporé dans l'article, ou dans lequel l'agent de décollement est initialement séparé de l'article et ensuite appliqué sur l'article.

**17.** Kit comprenant :
l'article multicouche selon l'une quelconque des revendications 1 à 16 ; et (b) un agent de décollement.

**18.** Kit selon la revendication 17 dans lequel l'agent de décollement comprend une silicone.

**19.** Kit selon la revendication 18 dans lequel la silicone possède la formule générale $(-CH_3)_2-SiO)_x$, dans laquelle x va de 2 à 300.

**20.** Kit selon l'une quelconque des revendications 17 à 19 comprenant en outre :

(c) un récipient dans lequel l'agent de décollement est stocké avant l'administration à l'article.

**21.** Kit selon l'une quelconque des revendications 17 à 20 dans lequel le kit est fourni dans un emballage unitaire.

**22.** Procédé d'aide au retrait d'un article à partir d'une surface après la fixation par adhérence de l'article sur celle-ci, dans lequel l'article comprend une feuille de support, au moins une couche adhésive disposée le long de la feuille de support, la couche adhésive comprenant un adhésif sensible à la pression comprenant (i) au moins un composant contenant une silicone, et (ii) au moins un polymère acrylique, une couche de substrat définissant au moins une ligne de découpe se prolongeant à travers son épaisseur, et une couche de régulation d'écoulement disposée entre l'au moins une couche adhésive et la couche de substrat, la couche de régulation d'écoulement présentant un profil d'écoulement régulable, le procédé comprenant :
l'exposition de l'article à une quantité efficace d'au moins un agent de décollement pour ainsi décoller l'article fixé par adhérence à partir de la surface.

**23.** Procédé selon la revendication 22 dans lequel l'exposition de l'article à une quantité efficace d'au moins un agent de décollement est réalisée par l'administration de l'agent de décollement à une région de la couche de régulation d'écoulement moyennant quoi l'agent de décollement est transporté jusqu'à au moins une autre région de la couche de régulation d'écoulement.

**24.** Procédé selon la revendication 23 moyennant quoi l'agent de décollement est transporté en outre jusqu'à une interface adhésive définie entre l'au moins une couche adhésive et la surface à laquelle l'article est fixé par adhérence.

**25.** Procédé pour appliquer par adhérence un article sur la peau et réduire la gêne associée au retrait de l'article à partir de la peau, le procédé comprenant :

la fourniture d'un article comprenant une feuille de support, au moins une couche adhésive disposée le long de la feuille de support, la couche adhésive comprenant un adhésif sensible à la pression comprenant (i) au moins un composant contenant une silicone, et (ii) au moins un polymère acrylique, une couche de substrat définissant au moins une ligne de découpe se prolongeant à travers son épaisseur, et une couche de régulation d'écoulement disposée entre l'au moins une couche adhésive et la couche de substrat, dans lequel la couche de régulation d'écoulement présente un profil d'écoulement régulable ;

l'application par adhérence de l'article sur la peau en mettant en contact au moins une partie de l'adhésif sensible à la pression sur la peau pour ainsi fixer par adhérence l'article sur la peau ;

l'exposition de l'article à une quantité efficace d'au moins un agent de décollement pour ainsi décoller l'article fixé par adhérence à partir de la peau.

26. Procédé selon la revendication 25 dans lequel l'exposition de l'article à une quantité efficace d'au moins un agent de décollement est réalisée par l'administration de l'agent de décollement à une région de la couche de régulation d'écoulement moyennant quoi l'agent de décollement est transporté jusqu'à au moins une autre région de la couche de régulation d'écoulement.

27. Procédé selon la revendication 26 moyennant quoi l'agent de décollement est transporté en outre jusqu'à une interface adhésive définie entre l'au moins une couche adhésive et la peau à laquelle l'article est fixé par adhérence.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

EP 2 877 135 B1

FIG. 16

FIG. 17

FIG. 18

FIG. 19

FIG. 20

FIG. 21

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55686712 A
- US 27063111 A
- US 02187311 A
- US 61302593 A
- US 4230838 A, Foy
- US 4493870 A, Vrouemraeto
- US 4725481 A, Ostapchenko
- US 3471445 A
- US 4532316 A
- US 5209969 A
- US 4194041 A
- US 5183841 A
- US 5385965 A

- US 2552520 A
- US 3998654 A
- US 4324595 A
- US 4867981 A
- US 5004502 A
- US 5336207 A
- US 6063231 A
- US 6436227 B
- US 7354889 B
- US 7396976 B
- US 7612248 B
- US 201155745 W
- WO 2010129299 A

**Non-patent literature cited in the description**

- **HEPBUM, C.** Polyurethane Elastomers. Applied Science Publishing Ltd, 1982
- Technology. **SAUNDERS S. FRISCH.** Polyurethanes chemistry and Technology. Robert E. Krieger Publishing Company, 1983

- **N. S. SCHNEIDER ; J. L. ILLINGER ; F. E. KARANSZ.** Hydrophilic polyurethane polymers are also commercially available in various forms - as solid resin, as a reactive prepolymer and as a solution in organic solvent or solvent mixtures. *Journal of Applied Polymer Science,* vol. 47, 1419